# EUROPEAN PATENT APPLICATION

(11) **EP 3 770 260 A1**
(43) Date of publication of application: **27.01.2021**
(21) Application number: 19771721.8
(22) Date of filing: 19.03.2019
(51) Int. Cl.: C12N 15/12, C07K 1/12, C07K 14/475, C12N 15/82, C12P 21/02, C12N 5/10

(54) **RECOMBINANT PRO-ACTIVIN A PROTEIN**

(30) Priority: 22.03.2018 JP 2018053947
(71) Applicant: AJINOMOTO CO., INC., Chuo-ku Tokyo 104-8315 (JP)
(72) Inventor: HIRANO, Hiroto, Kawasaki-shi, Kanagawa 210-8681 (JP); ITO, Kenichiro, Kawasaki-shi, Kanagawa 210-8681 (JP)
(74) Representative: Hoffmann Eitle
(86) International application number: PCT/JP2019/011357
(87) International publication number: WO 2019/181910

(57) **Abstract**

Provided is a recombinant activin A precursor protein that has human activin A activity. Also provided is a novel method for producing human activin A. The present invention pertains to a recombinant activin A precursor protein comprising a human mature activin A sequence and a non-human activin A prodomain, wherein said recombinant activin A precursor protein is not a naturally occurring activin A precursor protein. The present invention also pertains to a method for producing a human activin A using the aforesaid recombinant activin A precursor protein.

SEQUENCE LISTING

<110>  AJINOMOTO CO., INC.

<120>  RECOMBINANT ACTIVIN A PRECURSOR PROTEIN

<130>  OP19012

<150>  JP 2018-53947
<151>  2018-03-22

<160>  16

<170>  PatentIn version 3.5

<210>  1
<211>  10
<212>  PRT
<213>  Artificial Sequence

<220>
<223>  Common sequence

<400>  1

Asp Thr Arg Arg Ser Gly Trp His Thr Leu
1               5                   10


<210>  2
<211>  262
<212>  PRT
<213>  Activin A prodomain of Danio rerio

<400>  2

Ser Pro Thr Pro Ser Asp Ser Gly Ser Pro Gly Ala Gly Arg Pro Asp
1               5                   10                  15


Asp Pro Val Thr Pro Cys Pro Ser Cys Ala Leu Ala Gln Arg Pro Lys
            20                  25                  30


Asp Ser Glu Glu Gln Ser Asp Met Val Glu Ala Val Lys Gln His Ile
        35                  40                  45


Leu Asn Met Leu His Leu Asn Thr Arg Pro Asn Val Thr His Pro Val
    50                  55                  60


Pro Arg Ala Ala Leu Leu Asn Ala Ile Arg Lys Pro His Val Gly Arg
65                  70                  75                  80


Val Gly Glu Asp Gly Thr Val Glu Met Glu Glu Asp Gly Gly Gly Leu
                85                  90                  95


Gly Glu His Arg Glu Gln Pro Glu Glu Gln Pro Phe Glu Ile Ile Thr
            100                 105                 110

Phe Ala Glu Pro Gly Asp Ala Pro Asp Val Leu Lys Phe Asp Ile Ser
        115                 120                 125

Lys Glu Gly Ser Thr Leu Ser Val Val Glu Gln Ala Asn Val Trp Leu
    130                 135                 140

Phe Leu Lys Val Ala Lys Gly Ser Arg Val Lys Gly Lys Val Ser Val
145                 150                 155                 160

Gln Leu Leu Gln Asn Gly Lys Ala Asp Ser Gly Ser Thr Asp Arg Pro
                165                 170                 175

Glu Asp Gln Val Val Ser Glu Lys Thr Ile Asp Thr Arg Arg Ser Gly
            180                 185                 190

Trp His Thr Leu Pro Val Pro Arg Thr Val Gln Thr Leu Leu Asp Gly
        195                 200                 205

Asp Ser Ser Leu Leu Ser Leu Arg Val Ser Cys Pro Met Cys Ala Glu
    210                 215                 220

Ala Gly Ala Val Pro Ile Leu Val Pro Ala Glu Gly Asn Lys Val Lys
225                 230                 235                 240

Glu Arg Glu Gln Ser His Arg Pro Phe Leu Met Val Val Leu Lys Pro
                245                 250                 255

Ala Glu Glu His Gln His
            260

<210>  3
<211>  270
<212>  PRT
<213>  Activin A prodomain of Ictalurus punctatus

<400>  3

Ser Pro Thr Pro Ser Gly Met Gln Gly Thr Thr Pro Leu Glu Ser Glu
1               5                   10                  15

Arg Gln Glu Gly Ser Arg Gly Ala Glu Gln Gln Glu Glu Gly Gly Pro
            20                  25                  30

Val Ser Pro Cys Pro Ser Cys Ala Leu Ala Gln Leu Asp Arg Asp Ser
        35                  40                  45

Glu Pro Gly Met Val Glu Ala Val Lys Arg His Ile Leu Asn Met Leu
    50                  55                  60

His Leu Ser Ala Pro Pro Asn Ile Ser His Pro Val Pro Arg Ala Ala
65                  70                  75                  80

Leu Leu Asn Ala Leu Arg Lys Leu His Val Gly Arg Val Ala Gln Asp
                85                  90                  95

Gly Thr Val Glu Ile Glu Asp Glu Ala Glu Asp Phe Gly Gly His Ser
            100                 105                 110

Ala Gly Asp Glu Gln Pro Ser Glu Ile Ile Thr Phe Ala Glu Pro Val
        115                 120                 125

Asp Val Pro Asp Thr Val Lys Phe Asp Ile Ser Lys Glu Ser Ala Gly
    130                 135                 140

Gln Val Val Glu Gln Ala Asn Val Trp Ile Phe Leu Lys Leu Ala Lys
145                 150                 155                 160

Gly Ser His Ala Lys Gly Lys Val Ser Leu Gln Leu Leu Gln Ser Pro
                165                 170                 175

Ser Val Ser Thr Asp Ser Asn Pro Asp Pro Gln Asp Glu Val Leu Val
            180                 185                 190

Ser Gln Lys Met Val Asp Ala Arg Arg Ser Gly Trp His Thr Leu Ser
        195                 200                 205

Ile Gly Ala Ser Ala Gln Ala Leu Leu Asp Arg Gly Gly Gly Glu Leu
    210                 215                 220

Arg Phe Arg Val Ser Cys Pro Leu Cys Ala Asp Val Gly Ala Val Pro
225                 230                 235                 240

Ile Leu Gly Glu Gly Lys Gly Lys Glu His Ser Gln Ser His Arg Pro
                245                 250                 255

Phe Leu Met Leu Val Leu Arg Pro Ala Glu Asp Arg Gln His
            260                 265                 270

<210>  4
<211>  247
<212>  PRT
<213>  Activin A prodomain of Latimeria chalumnae

<400>  4

Ser Pro Ala Leu Gly Val Glu Gly His Ser Ser Val Pro Glu Cys Ala
1               5                   10                  15

Ser Cys Ala Leu Ala Lys Leu Pro Lys Asp Thr Ser Ser Ser Pro Pro
            20                  25                  30

Ala Met Val Glu Ala Val Lys Lys His Ile Leu Asn Met Leu His Leu
        35                  40                  45

Lys Glu Arg Pro Asn Ile Thr Gln Ala Val Pro Arg Ala Ala Leu Leu
    50                  55                  60

Asn Ala Ile Lys Lys Leu His Val Gly Arg Val Gly Glu Asp Gly Asn
65                  70                  75                  80

Val Glu Ile Glu Asp Asp Ser Tyr Arg Arg Leu Glu Ala Thr Glu Met
                85                  90                  95

Ile Glu Gln Thr Ser Glu Ile Ile Thr Phe Ala Glu Ser Gly Ser Ser
            100                 105                 110

Gln Gly Leu Leu His Phe Gln Ile Ser Lys Glu Gly Asn Asp Leu Ser
        115                 120                 125

Val Val Glu Gln Ala Asn Ile Trp Leu Phe Leu Arg Leu Ser Lys Ser
    130                 135                 140

Asn Arg Ser Arg Ala Lys Val Thr Ile Arg Ile His Gln Lys His Arg
145                 150                 155                 160

Gly Ser Asn Gly Gln Asp Ile Glu Ser Val Ile Ser Glu Lys Ala Val
                165                 170                 175

Asp Thr Arg Arg Ser Gly Trp His Thr Leu Pro Val Ser Ser Ser Val
            180                 185                 190

Gln His Leu Leu Asp Gly Gly His Thr Ser Leu Asp Ile Arg Ile Ser
        195                 200                 205

Cys Ser Gln Cys Gln Glu Asn Gly Val Thr Pro Val Leu Val Glu Lys
    210                 215                 220

Glu Glu Lys Glu Gln Ser His Arg Pro Phe Leu Met Ile Leu Val Arg
225                 230                 235                 240

Gln Ser Asp Asp His Pro His
                245

<210>  5
<211>  231

<212>  PRT
<213>  Activin A prodomain of Xenopus laevis

<400>  5

Ser Pro Thr Pro Glu Pro Gly Cys Pro Ser Cys His Pro Pro Met Glu
1               5                   10                  15

Pro Glu Met Leu Glu Ala Val Lys Arg His Ile Leu Thr Leu Leu His
            20                  25                  30

Met Gln Asp Arg Pro Asn Ile Thr His Met Val Pro Arg Ala Ala Met
        35                  40                  45

Val Ser Ala Leu Arg Lys Leu His Ala Gly Arg Val Arg Glu Asp Gly
    50                  55                  60

Asn Leu Glu Ile Pro Asp Leu Asp Gly His Ser Leu Pro Pro Pro Gly
65                  70                  75                  80

His Ser Thr Glu Asn Ser Ala Glu Ile Ile Thr Phe Ala Glu Thr Asp
                85                  90                  95

Asp Val Thr Ala Ser Arg Val Arg Leu Ser Phe Thr Ile Ala Asn Glu
            100                 105                 110

Gly Asn Gln Asn Leu Phe Val Phe Gln Ser Asn Leu Trp Leu Tyr Leu
        115                 120                 125

Lys Leu Pro Glu Val Met Asp Lys Ser Arg Arg Lys Ile Arg Ile Lys
    130                 135                 140

Val His Phe Gln Asp Ala Phe Asn Pro Asp Lys Met Asn Met Val Glu
145                 150                 155                 160

Lys Lys Val Asp Ile Arg Arg Ser Gly Trp His Thr Phe Pro Leu Thr
                165                 170                 175

Glu Ala Ile Gln Ser Leu Phe Glu Glu Gly Glu Arg Arg Leu Asn Leu
            180                 185                 190

Glu Val Gln Cys Asp Gly Cys Gly Glu Tyr Ser Val Ile Pro Val Tyr
        195                 200                 205

Val Asp Pro Gly Glu Glu Ser His Arg Pro Phe Leu Val Val His Ala
    210                 215                 220

Arg Leu Ala Asp Asn Lys His
225                 230

<210>  6
<211>  271
<212>  PRT
<213>  Activin A prodomain of Chelonia mydas

<400>  6

His Ser Ser Val Thr Gly Cys Pro Ser Cys Ala Leu Ala Thr Leu Ser
1               5                   10                  15

Lys Asp Val Pro Ser Ser Gln Pro Glu Met Val Glu Ala Val Lys Lys
            20                  25                  30

His Ile Leu Asn Met Leu His Leu Arg Asp Arg Pro Asn Ile Thr Gln
        35                  40                  45

Pro Val Pro Lys Ala Ala Leu Leu Asn Ala Ile Lys Lys Leu His Val
    50                  55                  60

Gly Lys Val Gly Glu Asp Gly Tyr Val Glu Ile Glu Asp Asp Ile Gly
65                  70                  75                  80

Arg Arg Ala Glu Met Asn Glu Leu Val Glu Gln Thr Ser Glu Ile Ile
                85                  90                  95

Thr Phe Ala Glu Ser Gly Thr Ala Lys Lys Met Leu His Phe Glu Ile
            100                 105                 110

Ser Lys Glu Gly Ser Asp Leu Ser Val Val Glu His Ala Glu Val Trp
        115                 120                 125

Leu Phe Leu Lys Val Ser Lys Ala Asn Arg Ser Arg Thr Lys Val Thr
    130                 135                 140

Ile Arg Leu Tyr Gln Gln Gln Arg Gln Pro Lys Gly Asn Ser Glu Gly
145                 150                 155                 160

Ala Glu Glu Met Glu Asp Gly Glu Leu Lys Gly Asp Lys Ser Glu Asn
                165                 170                 175

Leu Ile Ser Glu Lys Met Val Asp Thr Arg Lys Ser Thr Trp His Ile
            180                 185                 190

Phe Pro Val Ser Ser Ser Val Gln Tyr Leu Leu Asp Gln Gly Lys Ser
        195                 200                 205

Ser Leu Asp Val Arg Ile Ala Cys Asp Gln Cys Gln Glu Thr Gly Ala
    210                 215                 220

Ser Leu Val Leu Leu Gly Lys Lys Lys Lys Lys Glu Asp Lys Glu Lys
225                 230                 235                 240

Glu Val Gly Glu Ser Thr Val Glu Glu Glu Lys Glu Gln Ser His Arg
                245                 250                 255

Pro Phe Leu Met Met Leu Ala Arg His Ser Asp Asp Arg Leu His
            260                 265                 270

<210>  7
<211>  1260
<212>  DNA
<213>  Artificial Sequence

<220>
<223>  Recombinant DNA including Activin A prodomain of Danio rerio

<400>  7
atggctaaga ccaacctttt cctgttcctg atcttcagcc tgctgctgag cctttcttct      60

gctcatcatc accatcacca ctacccttac gatgtgcctg attacgcttc tcctacccct     120

agcgattctg gttctcctgg tgctggtaga cctgatgatc ctgttactcc ttgtccttct     180

tgcgctcttg ctcagaggcc taaggattct gaggaacaga gcgatatggt tgaggctgtg     240

aagcagcaca tcctgaacat gcttcacctt aacaccaggc ctaatgtgac tcatcctgtt     300

ccaagggctg ctctgcttaa cgctattagg aagcctcacg ttggtagggt tggagaggat     360

ggtactgttg agatggaaga ggatggtggt ggtcttggtg aacatagaga acagcctgaa     420

gaacagccat tcgagatcat cactttcgct gagcctggtg atgctcctga tgtgctgaag     480

ttcgatatca gcaaagaggg tagcaccctg tctgttgttg agcaggctaa tgtgtggctt     540

ttcctgaagg tggcaaaggg ttctagggtg aagggtaagg ttagcgtgca gcttcttcag     600

aacggtaagg ctgatagcgg ttctactgat aggcctgagg atcaggtggt gagcgaaaag     660

accattgata ccagaaggtc tggttggcac actttgccag ttcctaggac tgtgcagacc     720

cttctggatg gtgatagctc tctgctttct ctgagggtgt catgtcctat gtgtgctgag     780

gctggtgctg tgcctattct tgttcctgct gagggtaaca aggtgaaaga gagagagcag     840

tctcacaggc ctttcttgat ggtggttttg aagcctgcag aggaacacca gcacagaagg     900

tctaagcgtg gacttgaatg tgatggtaag gttaatattt gctgtaaaaa gcaatttttc     960

gtttctttta aagatattgg atggaatgat tggattattg ctccatctgg ttatcatgct    1020

aattattgtg aaggagagtg tccttctcat attgctggta cttctggatc ttcattgtca    1080

tttcattcta ctgttattaa tcattataga atgaggggtc attctccatt tgctaatctt    1140

aagtcttgtt gtgttccaac taagttgaga ccaatgtcta tgctttacta tgatgatgga    1200

caaaatatta ttaaaaagga tattcaaaat atgattgttg aagagtgtgg atgctcttag     1260

<210>  8
<211>  419
<212>  PRT
<213>  Artificial Sequence

<220>
<223>  Recombinant Protein including Activin A prodomain of Danio rerio

<400>  8

Met Ala Lys Thr Asn Leu Phe Leu Phe Leu Ile Phe Ser Leu Leu Leu
1               5                   10                  15

Ser Leu Ser Ser Ala His His His His His His Tyr Pro Tyr Asp Val
            20                  25                  30

Pro Asp Tyr Ala Ser Pro Thr Pro Ser Asp Ser Gly Ser Pro Gly Ala
        35                  40                  45

Gly Arg Pro Asp Asp Pro Val Thr Pro Cys Pro Ser Cys Ala Leu Ala
    50                  55                  60

Gln Arg Pro Lys Asp Ser Glu Glu Gln Ser Asp Met Val Glu Ala Val
65                  70                  75                  80

Lys Gln His Ile Leu Asn Met Leu His Leu Asn Thr Arg Pro Asn Val
                85                  90                  95

Thr His Pro Val Pro Arg Ala Ala Leu Leu Asn Ala Ile Arg Lys Pro
            100                 105                 110

His Val Gly Arg Val Gly Glu Asp Gly Thr Val Glu Met Glu Glu Asp
        115                 120                 125

Gly Gly Gly Leu Gly Glu His Arg Glu Gln Pro Glu Glu Gln Pro Phe
    130                 135                 140

Glu Ile Ile Thr Phe Ala Glu Pro Gly Asp Ala Pro Asp Val Leu Lys
145                 150                 155                 160

Phe Asp Ile Ser Lys Glu Gly Ser Thr Leu Ser Val Val Glu Gln Ala
                165                 170                 175

Asn Val Trp Leu Phe Leu Lys Val Ala Lys Gly Ser Arg Val Lys Gly
            180                 185                 190

Lys Val Ser Val Gln Leu Leu Gln Asn Gly Lys Ala Asp Ser Gly Ser
        195                 200                 205

Thr Asp Arg Pro Glu Asp Gln Val Val Ser Glu Lys Thr Ile Asp Thr
    210                 215                 220

Arg Arg Ser Gly Trp His Thr Leu Pro Val Pro Arg Thr Val Gln Thr
225                 230                 235                 240

Leu Leu Asp Gly Asp Ser Ser Leu Leu Ser Leu Arg Val Ser Cys Pro
                245                 250                 255

Met Cys Ala Glu Ala Gly Ala Val Pro Ile Leu Val Pro Ala Glu Gly
            260                 265                 270

Asn Lys Val Lys Glu Arg Glu Gln Ser His Arg Pro Phe Leu Met Val
        275                 280                 285

Val Leu Lys Pro Ala Glu Glu His Gln His Arg Arg Ser Lys Arg Gly
    290                 295                 300

Leu Glu Cys Asp Gly Lys Val Asn Ile Cys Cys Lys Lys Gln Phe Phe
305                 310                 315                 320

Val Ser Phe Lys Asp Ile Gly Trp Asn Asp Trp Ile Ile Ala Pro Ser
                325                 330                 335

Gly Tyr His Ala Asn Tyr Cys Glu Gly Glu Cys Pro Ser His Ile Ala
            340                 345                 350

Gly Thr Ser Gly Ser Ser Leu Ser Phe His Ser Thr Val Ile Asn His
        355                 360                 365

Tyr Arg Met Arg Gly His Ser Pro Phe Ala Asn Leu Lys Ser Cys Cys
    370                 375                 380

Val Pro Thr Lys Leu Arg Pro Met Ser Met Leu Tyr Tyr Asp Asp Gly
385                 390                 395                 400

Gln Asn Ile Ile Lys Lys Asp Ile Gln Asn Met Ile Val Glu Glu Cys
                405                 410                 415

Gly Cys Ser

<210>  9
<211>  1284
<212>  DNA
<213>  Artificial Sequence

<220>
<223>  Recombinant DNA including Activin A prodomain of Ictalurus
       punctatus

<400>  9
atggctaaga ctaacctttt cctgttcctg atcttcagcc tgctgctgag cctttcttct      60

gctcatcatc accatcacca ctacccttac gatgtgcctg attacgcttc tccaactcca     120

tctggtatgc agggtactac ccctcttgag tctgaaaggc aagagggttc tagaggtgct     180

gagcaacaag aggaaggtgg tcctgtttct ccttgtcctt cttgtgctct tgctcagctg     240

gatagggatt ctgagcctgg tatggttgag gctgtgaaga ggcacattct gaacatgctt     300

cacctgagcg ctcctccgaa tatttctcat cctgttccaa gggctgctct gctgaacgct     360

cttagaaagc ttcatgtggg tagagtggct caggatggca ctgttgaaat tgaggatgag     420

gctgaggatt tcggtggtca ctctgctggt gatgaacagc cttctgagat catcactttc     480

gctgagcctg tggatgtgcc tgataccgtg aagttcgaca tcagcaaaga gagcgctggc     540

caagttgttg agcaggctaa tgtgtggatc ttcctgaagc tggctaaggg ctctcacgct     600

aagggtaaag tgtctcttca gctcctgcag tccccttctg tgtctactga ttctaaccct     660

gatccgcagg atgaggtgct ggtgtctcaa aagatggtgg atgctagaag gtctggttgg     720

cacacccttt ctattggtgc ttcagctcag gctctgcttg atagaggtgg tggtgagctt     780

aggttcagag tgtcttgtcc tctttgcgct gatgttggtg ctgtgcctat tcttggtgaa     840

ggcaaggaaa aagagcacag ccagtctcat aggccgttcc ttatgcttgt tcttaggcct     900

gctgaggaca ggcaacatag aaggtctaag cgtggacttg aatgtgatgg taaggttaat     960

atttgctgta aaaagcaatt tttcgtttct tttaaagata ttggatggaa tgattggatt    1020

attgctccat ctggttatca tgctaattat tgtgaaggag agtgtccttc tcatattgct    1080

ggtacttctg gatcttcatt gtcatttcat tctactgtta ttaatcatta tagaatgagg    1140

ggtcattctc catttgctaa tcttaagtct tgttgtgttc caactaagtt gagaccaatg    1200

tctatgcttt actatgatga tggacaaaat attattaaaa aggatattca aaatatgatt    1260

gttgaagagt gtggatgctc ttag                                           1284


<210>  10
<211>  427
<212>  PRT
<213>  Artificial Sequence

<220>
<223>  Recombinant Protein including Activin A prodomain of Ictalurus
       punctatus

<400>  10

Met Ala Lys Thr Asn Leu Phe Leu Phe Leu Ile Phe Ser Leu Leu Leu
1               5                   10                  15

Ser Leu Ser Ser Ala His His His His His His Tyr Pro Tyr Asp Val
            20                  25                  30

Pro Asp Tyr Ala Ser Pro Thr Pro Ser Gly Met Gln Gly Thr Thr Pro
        35                  40                  45

Leu Glu Ser Glu Arg Gln Glu Gly Ser Arg Gly Ala Glu Gln Gln Glu
    50                  55                  60

Glu Gly Gly Pro Val Ser Pro Cys Pro Ser Cys Ala Leu Ala Gln Leu
65                  70                  75                  80

Asp Arg Asp Ser Glu Pro Gly Met Val Glu Ala Val Lys Arg His Ile
                85                  90                  95

Leu Asn Met Leu His Leu Ser Ala Pro Pro Asn Ile Ser His Pro Val
            100                 105                 110

Pro Arg Ala Ala Leu Leu Asn Ala Leu Arg Lys Leu His Val Gly Arg
        115                 120                 125

Val Ala Gln Asp Gly Thr Val Glu Ile Glu Asp Glu Ala Glu Asp Phe
    130                 135                 140

Gly Gly His Ser Ala Gly Asp Glu Gln Pro Ser Glu Ile Ile Thr Phe
145                 150                 155                 160

Ala Glu Pro Val Asp Val Pro Asp Thr Val Lys Phe Asp Ile Ser Lys
                165                 170                 175

Glu Ser Ala Gly Gln Val Val Glu Gln Ala Asn Val Trp Ile Phe Leu
            180                 185                 190

Lys Leu Ala Lys Gly Ser His Ala Lys Gly Lys Val Ser Leu Gln Leu
        195                 200                 205

Leu Gln Ser Pro Ser Val Ser Thr Asp Ser Asn Pro Asp Pro Gln Asp
    210                 215                 220

Glu Val Leu Val Ser Gln Lys Met Val Asp Ala Arg Arg Ser Gly Trp
225                 230                 235                 240

His Thr Leu Ser Ile Gly Ala Ser Ala Gln Ala Leu Leu Asp Arg Gly
                245                 250                 255

Gly Gly Glu Leu Arg Phe Arg Val Ser Cys Pro Leu Cys Ala Asp Val

260                 265                 270

Gly Ala Val Pro Ile Leu Gly Glu Gly Lys Gly Lys Glu His Ser Gln
        275                 280                 285

Ser His Arg Pro Phe Leu Met Leu Val Leu Arg Pro Ala Glu Asp Arg
    290                 295                 300

Gln His Arg Arg Ser Lys Arg Gly Leu Glu Cys Asp Gly Lys Val Asn
305                 310                 315                 320

Ile Cys Cys Lys Lys Gln Phe Phe Val Ser Phe Lys Asp Ile Gly Trp
                325                 330                 335

Asn Asp Trp Ile Ile Ala Pro Ser Gly Tyr His Ala Asn Tyr Cys Glu
            340                 345                 350

Gly Glu Cys Pro Ser His Ile Ala Gly Thr Ser Gly Ser Ser Leu Ser
        355                 360                 365

Phe His Ser Thr Val Ile Asn His Tyr Arg Met Arg Gly His Ser Pro
    370                 375                 380

Phe Ala Asn Leu Lys Ser Cys Cys Val Pro Thr Lys Leu Arg Pro Met
385                 390                 395                 400

Ser Met Leu Tyr Tyr Asp Asp Gly Gln Asn Ile Ile Lys Lys Asp Ile
                405                 410                 415

Gln Asn Met Ile Val Glu Glu Cys Gly Cys Ser
            420                 425

<210>  11
<211>  1215
<212>  DNA
<213>  Artificial Sequence

<220>
<223>  Recombinant DNA including Activin A prodomain of Latimeria
       chalumnae

<400>  11
atggctaaga ctaacctttt cctgttcctg atcttcagcc tgctgctgag cctttcttct      60

gctcatcatc accaccatca ctacccttac gatgtgcctg attacgcttc tcctgctctt     120

ggtgttgagg gacattcttc tgttccagag tgtgcttctt gcgctcttgc taagcttccg     180

aaggatacct cttctagccc tcctgctatg gttgaggctg tgaagaaaca catcctgaac     240

atgctgcacc tgaaagagag gcctaacatt actcaggctg ttcctagggc tgctctgctg     300

aacgctatta agaagctgca cgttggtaga gttggcgagg atggtaacgt tgagatcgag 360

gatgatagct acaggcggct tgaagctacc gagatgattg aacagaccag cgagatcatc 420

accttcgctg agtctggttc ttctcagggt ttgctgcact tccagatcag caaagagggc 480

aacgatctgt ctgttgtcga gcaggctaac atctggcttt ttctgcggct gagcaagagc 540

aacaggtcta gagctaaggt gaccatccgg attcaccaaa agcacagggg ttctaacggt 600

caggatatcg agtctgtgat cagcgagaag gctgtggata caagaaggtc tggttggcac 660

actctcccgg tttcttctag tgtgcagcac cttcttgatg gtggccacac ctctcttgac 720

atcaggattt cttgcagcca gtgccaagag aatggtgtga ctcctgtgct ggtcgagaaa 780

gaagagaaag agcagtctca ccggccgttc cttatgattc ttgtgaggca gtctgacgat 840

caccctcaca gacgtaagaa acggggactt gaatgtgatg gtaaggttaa tatttgctgt 900

aaaaagcaat ttttcgtttc ttttaaagat attggatgga atgattggat tattgctcca 960

tctggttatc atgctaatta ttgtgaagga gagtgtcctt ctcatattgc tggtacttct 1020

ggatcttcat tgtcatttca ttctactgtt attaatcatt atagaatgag gggtcattct 1080

ccatttgcta atcttaagtc ttgttgtgtt ccaactaagt tgagaccaat gtctatgctt 1140

tactatgatg atggacaaaa tattattaaa aaggatattc aaaatatgat tgttgaagag 1200

tgtggatgct cttag 1215

<210> 12
<211> 404
<212> PRT
<213> Artificial Sequence

<220>
<223> Recombinant Protein including Activin A prodomain of Latimeria
 chalumnae

<400> 12

Met Ala Lys Thr Asn Leu Phe Leu Phe Leu Ile Phe Ser Leu Leu Leu
1               5                   10                  15

Ser Leu Ser Ser Ala His His His His His His Tyr Pro Tyr Asp Val
            20                  25                  30

Pro Asp Tyr Ala Ser Pro Ala Leu Gly Val Glu Gly His Ser Ser Val
        35                  40                  45

Pro Glu Cys Ala Ser Cys Ala Leu Ala Lys Leu Pro Lys Asp Thr Ser
    50                  55                  60

Ser Ser Pro Pro Ala Met Val Glu Ala Val Lys Lys His Ile Leu Asn
65                  70                  75                  80

Met Leu His Leu Lys Glu Arg Pro Asn Ile Thr Gln Ala Val Pro Arg
                85                  90                  95

Ala Ala Leu Leu Asn Ala Ile Lys Lys Leu His Val Gly Arg Val Gly
            100                 105                 110

Glu Asp Gly Asn Val Glu Ile Glu Asp Asp Ser Tyr Arg Arg Leu Glu
        115                 120                 125

Ala Thr Glu Met Ile Glu Gln Thr Ser Glu Ile Ile Thr Phe Ala Glu
    130                 135                 140

Ser Gly Ser Ser Gln Gly Leu Leu His Phe Gln Ile Ser Lys Glu Gly
145                 150                 155                 160

Asn Asp Leu Ser Val Val Glu Gln Ala Asn Ile Trp Leu Phe Leu Arg
                165                 170                 175

Leu Ser Lys Ser Asn Arg Ser Arg Ala Lys Val Thr Ile Arg Ile His
            180                 185                 190

Gln Lys His Arg Gly Ser Asn Gly Gln Asp Ile Glu Ser Val Ile Ser
        195                 200                 205

Glu Lys Ala Val Asp Thr Arg Arg Ser Gly Trp His Thr Leu Pro Val
    210                 215                 220

Ser Ser Ser Val Gln His Leu Leu Asp Gly Gly His Thr Ser Leu Asp
225                 230                 235                 240

Ile Arg Ile Ser Cys Ser Gln Cys Gln Glu Asn Gly Val Thr Pro Val
                245                 250                 255

Leu Val Glu Lys Glu Glu Lys Glu Gln Ser His Arg Pro Phe Leu Met
            260                 265                 270

Ile Leu Val Arg Gln Ser Asp Asp His Pro His Arg Arg Lys Lys Arg
        275                 280                 285

Gly Leu Glu Cys Asp Gly Lys Val Asn Ile Cys Cys Lys Lys Gln Phe
    290                 295                 300

Phe Val Ser Phe Lys Asp Ile Gly Trp Asn Asp Trp Ile Ile Ala Pro
305                 310                 315                 320

Ser Gly Tyr His Ala Asn Tyr Cys Glu Gly Glu Cys Pro Ser His Ile
                325                 330                 335

Ala Gly Thr Ser Gly Ser Ser Leu Ser Phe His Ser Thr Val Ile Asn
            340                 345                 350

His Tyr Arg Met Arg Gly His Ser Pro Phe Ala Asn Leu Lys Ser Cys
        355                 360                 365

Cys Val Pro Thr Lys Leu Arg Pro Met Ser Met Leu Tyr Tyr Asp Asp
    370                 375                 380

Gly Gln Asn Ile Ile Lys Lys Asp Ile Gln Asn Met Ile Val Glu Glu
385                 390                 395                 400

Cys Gly Cys Ser

<210>  13
<211>  1167
<212>  DNA
<213>  Artificial Sequence

<220>
<223>  Recombinant DNA including Activin A prodomain of Xenopus laevis

<400>  13
atggctaaga ccaacctttt cctgttcctg atcttcagcc tgctgctgag cctttcttct      60

gctcatcatc accatcacca ctacccttac gatgtgcctg attacgcttc tcctactcct     120

gaacctggtt gcccttcttg tcatcctcct atggaacctg agatgcttga ggctgtgaag     180

aggcacattc ttaccctgct tcacatgcag gataggccta acattaccca catggttcct     240

agggctgcta tggtgtctgc tcttagaaag cttcatgctg gtagggtgag agaggatggt     300

aaccttgaga tccctgatct ggatggtcac tctttgcctc cacctggtca ttctactgag     360

aacagcgctg agatcatcac cttcgctgag actgatgatg tgaccgcttc tagggtgagg     420

ctgtctttca ctattgctaa cgagggtaac cagaacctgt tcgtgttcca gtctaacctg     480

tggctgtacc ttaagctgcc tgaggtgatg gataagtcta gaagaaagat taggatcaag     540

gtgcacttcc aggatgcttt caaccctgat aagatgaaca tggttgagaa gaaagtggat     600

atcagaaggt ccggttggca cacctttcca cttactgagg ctatccagtc cctgttcgaa     660

gaaggtgaga gaaggcttaa cttggaggtg cagtgtgatg gttgcggtga gtactctgtg     720

atccctgtgt atgttgatcc tggtgaagag agccacaggc ctttcttggt tgttcatgct     780

aggctggctg ataacaagca ccggatccgt aagaggggac ttgaatgtga tggtaaggtt     840

aatatttgct gtaaaaagca atttttcgtt tcttttaaag atattggatg gaatgattgg     900

attattgctc catctggtta tcatgctaat tattgtgaag gagagtgtcc ttctcatatt     960

gctggtactt ctggatcttc attgtcattt cattctactg ttattaatca ttatagaatg 1020

aggggtcatt ctccatttgc taatcttaag tcttgttgtg ttccaactaa gttgagacca 1080

atgtctatgc tttactatga tgatggacaa aatattatta aaaaggatat tcaaaatatg 1140

attgttgaag agtgtggatg ctcttaa 1167

<210> 14
<211> 388
<212> PRT
<213> Artificial Sequence

<220>
<223> Recombinant Protein including Activin A prodomain of Xenopus
      laevis

<400> 14

Met Ala Lys Thr Asn Leu Phe Leu Phe Leu Ile Phe Ser Leu Leu Leu
1               5                   10                  15

Ser Leu Ser Ser Ala His His His His His His Tyr Pro Tyr Asp Val
            20                  25                  30

Pro Asp Tyr Ala Ser Pro Thr Pro Glu Pro Gly Cys Pro Ser Cys His
        35                  40                  45

Pro Pro Met Glu Pro Glu Met Leu Glu Ala Val Lys Arg His Ile Leu
    50                  55                  60

Thr Leu Leu His Met Gln Asp Arg Pro Asn Ile Thr His Met Val Pro
65                  70                  75                  80

Arg Ala Ala Met Val Ser Ala Leu Arg Lys Leu His Ala Gly Arg Val
                85                  90                  95

Arg Glu Asp Gly Asn Leu Glu Ile Pro Asp Leu Asp Gly His Ser Leu
            100                 105                 110

Pro Pro Pro Gly His Ser Thr Glu Asn Ser Ala Glu Ile Ile Thr Phe
        115                 120                 125

Ala Glu Thr Asp Asp Val Thr Ala Ser Arg Val Arg Leu Ser Phe Thr
    130                 135                 140

Ile Ala Asn Glu Gly Asn Gln Asn Leu Phe Val Phe Gln Ser Asn Leu
145                 150                 155                 160

Trp Leu Tyr Leu Lys Leu Pro Glu Val Met Asp Lys Ser Arg Arg Lys
                165                 170                 175

Ile Arg Ile Lys Val His Phe Gln Asp Ala Phe Asn Pro Asp Lys Met
            180                 185                 190

Asn Met Val Glu Lys Lys Val Asp Ile Arg Arg Ser Gly Trp His Thr
        195                 200                 205

Phe Pro Leu Thr Glu Ala Ile Gln Ser Leu Phe Glu Glu Gly Glu Arg
    210                 215                 220

Arg Leu Asn Leu Glu Val Gln Cys Asp Gly Cys Gly Glu Tyr Ser Val
225                 230                 235                 240

Ile Pro Val Tyr Val Asp Pro Gly Glu Glu Ser His Arg Pro Phe Leu
                245                 250                 255

Val Val His Ala Arg Leu Ala Asp Asn Lys His Arg Ile Arg Lys Arg
            260                 265                 270

Gly Leu Glu Cys Asp Gly Lys Val Asn Ile Cys Cys Lys Lys Gln Phe
        275                 280                 285

Phe Val Ser Phe Lys Asp Ile Gly Trp Asn Asp Trp Ile Ile Ala Pro
    290                 295                 300

Ser Gly Tyr His Ala Asn Tyr Cys Glu Gly Glu Cys Pro Ser His Ile
305                 310                 315                 320

Ala Gly Thr Ser Gly Ser Ser Leu Ser Phe His Ser Thr Val Ile Asn
                325                 330                 335

His Tyr Arg Met Arg Gly His Ser Pro Phe Ala Asn Leu Lys Ser Cys
            340                 345                 350

Cys Val Pro Thr Lys Leu Arg Pro Met Ser Met Leu Tyr Tyr Asp Asp
        355                 360                 365

Gly Gln Asn Ile Ile Lys Lys Asp Ile Gln Asn Met Ile Val Glu Glu
    370                 375                 380

Cys Gly Cys Ser
385

<210> 15
<211> 1260
<212> DNA
<213> Artificial Sequence

<220>

<223>  Recombinant DNA including Activin A prodomain of Chelonia mydas

<400>  15
atggctaaga ccaacctttt cctgttcctg atcttcagcc tgctgctgag cctttcttct      60

gctcatcatc accatcacca tcattcttct gtgaccggtt gcccttcttg tgctcttgct     120

accctttcta aggatgtgcc atctagccag cctgagatgg ttgaggctgt gaagaaacac     180

atcctgaaca tgctgcacct gagggatagg cctaatatta ctcagcctgt gcctaaggct     240

gctctgctga acgctattaa gaagctgcac gttggtaagg tgggagagga tggttacgtt     300

gagatcgagg atgatatcgg tagaagggct gagatgaacg agcttgttga gcagacctct     360

gagatcatca ccttcgctga atctggaacc gctaagaaga tgctgcactt cgagatcagc     420

aaagagggta gcgatctgtc tgtggttgag catgctgagg tgtggctttt cctgaaggtg     480

tcaaaggcta acaggtccag gaccaaggtg accattaggc tttatcaaca acagaggcag     540

cctaagggaa actctgaagg tgctgaagag atggaagatg gtgagctgaa gggtgataag     600

agcgagaacc tgatcagcga aaagatggtg gataccagaa agagcacctg gcacatcttc     660

cctgtgtcat cttctgtgca gtacctgctg gatcagggta agagttctct ggatgtgagg     720

atcgcttgcg atcagtgtca agagactggt gcttctcttg tgctgctggg taagaagaag     780

aaaaaagagg ataaagagaa agaggttgga gagtccaccg ttgaggaaga aaaagagcag     840

tctcacaggc ctttcttgat gatgcttgct aggcactccg atgataggct tcaccgtaga     900

cgtagacgtg gacttgaatg tgatggtaag gttaatattt gctgtaaaaa gcaatttttc     960

gtttctttta aagatattgg atggaatgat tggattattg ctccatctgg ttatcatgct    1020

aattattgtg aaggagagtg tccttctcat attgctggta cttctggatc ttcattgtca    1080

tttcattcta ctgttattaa tcattataga atgaggggtc attctccatt tgctaatctt    1140

aagtcttgtt gtgttccaac taagttgaga ccaatgtcta tgctttacta tgatgatgga    1200

caaaatatta ttaaaaagga tattcaaaat atgattgttg aagagtgtgg atgctcttag    1260


<210>  16
<211>  419
<212>  PRT
<213>  Artificial Sequence

<220>
<223>  Recombinant Protein including Activin A prodomain of Chelonia
       mydas

<400>  16
   Met Ala Lys Thr Asn Leu Phe Leu Phe Leu Ile Phe Ser Leu Leu Leu
   1               5                   10                  15
   Ser Leu Ser Ser Ala His His His His His His His Ser Ser Val Thr
               20                  25                  30
   Gly Cys Pro Ser Cys Ala Leu Ala Thr Leu Ser Lys Asp Val Pro Ser
           35                  40                  45
   Ser Gln Pro Glu Met Val Glu Ala Val Lys Lys His Ile Leu Asn Met
       50                  55                  60
   Leu His Leu Arg Asp Arg Pro Asn Ile Thr Gln Pro Val Pro Lys Ala
   65                  70                  75                  80
   Ala Leu Leu Asn Ala Ile Lys Lys Leu His Val Gly Lys Val Gly Glu
                   85                  90                  95
   Asp Gly Tyr Val Glu Ile Glu Asp Asp Ile Gly Arg Arg Ala Glu Met
               100                 105                 110
   Asn Glu Leu Val Glu Gln Thr Ser Glu Ile Ile Thr Phe Ala Glu Ser
           115                 120                 125
   Gly Thr Ala Lys Lys Met Leu His Phe Glu Ile Ser Lys Glu Gly Ser
       130                 135                 140
   Asp Leu Ser Val Val Glu His Ala Glu Val Trp Leu Phe Leu Lys Val
   145                 150                 155                 160
   Ser Lys Ala Asn Arg Ser Arg Thr Lys Val Thr Ile Arg Leu Tyr Gln
                   165                 170                 175
   Gln Gln Arg Gln Pro Lys Gly Asn Ser Glu Gly Ala Glu Glu Met Glu
               180                 185                 190
   Asp Gly Glu Leu Lys Gly Asp Lys Ser Glu Asn Leu Ile Ser Glu Lys
           195                 200                 205
   Met Val Asp Thr Arg Lys Ser Thr Trp His Ile Phe Pro Val Ser Ser
       210                 215                 220
   Ser Val Gln Tyr Leu Leu Asp Gln Gly Lys Ser Ser Leu Asp Val Arg
   225                 230                 235                 240
   Ile Ala Cys Asp Gln Cys Gln Glu Thr Gly Ala Ser Leu Val Leu Leu
                   245                 250                 255
   Gly Lys Lys Lys Lys Lys Glu Asp Lys Glu Lys Glu Val Gly Glu Ser
               260                 265                 270
   Thr Val Glu Glu Glu Lys Glu Gln Ser His Arg Pro Phe Leu Met Met
   275 280 285
   Leu Ala Arg His Ser Asp Asp Arg Leu His Arg Arg Arg Arg Arg Gly
   290 295 300
   Leu Glu Cys Asp Gly Lys Val Asn Ile Cys Cys Lys Lys Gln Phe Phe
   305 310 315 320
   Val Ser Phe Lys Asp Ile Gly Trp Asn Asp Trp Ile Ile Ala Pro Ser
   325 330 335
   Gly Tyr His Ala Asn Tyr Cys Glu Gly Glu Cys Pro Ser His Ile Ala
   340 345 350
   Gly Thr Ser Gly Ser Ser Leu Ser Phe His Ser Thr Val Ile Asn His
   355 360 365
   Tyr Arg Met Arg Gly His Ser Pro Phe Ala Asn Leu Lys Ser Cys Cys
   370 375 380
   Val Pro Thr Lys Leu Arg Pro Met Ser Met Leu Tyr Tyr Asp Asp Gly
   385 390 395 400
   Gln Asn Ile Ile Lys Lys Asp Ile Gln Asn Met Ile Val Glu Glu Cys
   405 410 415
   Gly Cys Ser

## Description

### Technical Field

The present invention relates to a recombinant activin A precursor protein, a method for producing human activin A using the same, and the like.

### Background Art

Proteins belonging to the TGF-β family contribute to the generation of living organisms, differentiation, adjustment of cell proliferation, and the like, and are also important as differentiating factors for undifferentiated cells. However, since these proteins have complicated disulfide bonds in the molecules, it is often the case that when these proteins are produced using *Escherichia coli* and the like, the proteins are not correctly folded and fail to reproduce the conformations, thus having no activity.

Activin A, which belongs to the TGF-β family, is a protein present as a homodimer in which two polypeptide chains are linked by multiple disulfide bonds. Activin A is expressed in the state of a precursor (also referred to as pro-activin A), and the precursor has a mature activin A sequence and an activin A prodomain. It is known that in the state of the precursor (pro-activin A), activin A has no activity, and by cleaving the prodomain portion, the mature activin A sequence is released and forms active activin A having a predetermined conformation (Xuelu Wang et al., Nature Communications, 2016, 7:12052). In addition, activin A is known to induce iPS cells to differentiate into definitive endoderm.

Although there have been attempts to produce human activin A by utilizing microorganisms such as *Escherichia coli* as hosts, human activin A thus produced is often not correctly folded, and fails to reproduce the conformation, thus having no activity. For this reason, it is difficult to produce human activin A having activity by utilizing microorganisms such as *Escherichia coli* as hosts.

A method for refolding the conformation of human activin A having no activity into the conformation of human activin A having activity has been known (International Publication No. 97/23638). However, since it is necessary to conduct the refolding method separately after producing human activin A utilizing microorganisms such as *Escherichia coli* as hosts, this method cannot be said to be simple and convenient.

In addition, as described above, it is known that in the state of the precursor, activin A has no activity, and by cleaving the prodomain portion, the mature activin A sequence is released and forms active activin A. No activin A precursor protein having activity while having the prodomain portion has been known so far.

### Summary of Invention

An object of one aspect of the present invention is to provide a novel method for producing human activin A.

In addition, an object of another aspect of the present invention is to provide a recombinant activin A precursor protein for use in the novel method for producing human activin A.

Furthermore, an object of still another aspect of the present invention is to provide a recombinant activin A precursor protein having human activin A activity.

The present inventors attempted to produce human activin A by using plant cells. However, the accumulation of target human activin A was not confirmed merely by expressing a gene encoding only a human mature activin A sequence forming human activin A having activity in plant cells. Even for animals that express activin A, it has been originally impossible to express only the active type (mature activin sequence), and it was found to be significantly difficult to express only the active type (mature activin sequence).

As a result of earnest studies, the present inventors newly found that it is possible to produce human activin A having activity by expressing, in plant cells, not a gene encoding only a human mature activin A sequence, but a gene encoding a recombinant activin A precursor protein comprising a human mature activin A sequence and a non-human activin A prodomain to generate the recombinant activin A precursor protein, and subsequently cleaving the human mature activin A sequence from the recombinant activin A precursor protein.

In addition, the present inventors newly found that the recombinant activin A precursor protein comprising the human mature activin A sequence and the non-human activin A prodomain has human activin A activity by itself.

The present invention has been made based on these new findings.

The present invention may include, for example, the following aspects:
[1] A recombinant activin A precursor protein comprising:
   a human mature activin A sequence; and
   a non-human activin A prodomain, wherein
   the recombinant activin A precursor protein is not a naturally occurring activin A precursor protein.
[2] The recombinant activin A precursor protein according to [1], wherein
   the non-human activin A prodomain is an activin A prodomain of an amphibian, a reptile, or a fish.
[3] The recombinant activin A precursor protein according to [1] or [2], wherein
   the non-human activin A prodomain is an activin A prodomain of a fish.
[4] The recombinant activin A precursor protein according to [3], wherein
   the activin A prodomain of a fish comprises a sequence having an amino acid sequence identity of 90% or more with a sequence represented by SEQ ID NO: 1.
[5] The recombinant activin A precursor protein according to [4], wherein
   the activin A prodomain of a fish has the sequence having an amino acid sequence identity of 90% or more with the sequence represented by SEQ ID NO: 1 at positions corresponding to positions 187 to 196 of a sequence represented by SEQ ID NO: 2.
[6] The recombinant activin A precursor protein according to any one of [1] to [5], wherein
   the non-human activin A prodomain has an amino acid sequence identity of 90% or more with a sequence represented by SEQ ID NO: 2.
[7] The recombinant activin A precursor protein according to any one of [1] to [5], wherein
   the non-human activin A prodomain has an amino acid sequence identity of 90% or more with a sequence represented by SEQ ID NO: 3.
[8] The recombinant activin A precursor protein according to any one of [1] to [5], wherein
   the non-human activin A prodomain has an amino acid sequence identity of 90% or more with a sequence represented by SEQ ID NO: 4.
[9] The recombinant activin A precursor protein according to [1] or [2], wherein
   the non-human activin A prodomain has an amino acid sequence identity of 90% or more with a sequence represented by SEQ ID NO: 5.
[10] The recombinant activin A precursor protein according to [1] or [2], wherein
   the non-human activin A prodomain has an amino acid sequence identity of 90% or more with a sequence represented by SEQ ID NO: 6.
[11] The recombinant activin A precursor protein according to any one of [1] to [10], comprising:
   a sequence specifically cleavable by a protease between the human mature activin A sequence and the non-human activin A prodomain.
[12] A polynucleotide encoding the recombinant activin A precursor protein according to any one of [1] to [11].
[13] A recombinant vector comprising:
   the polynucleotide according to [12].
[14] A transformant comprising:
   the polynucleotide according to [12] or the recombinant vector according to [13].
[15] A method for producing the recombinant activin A precursor protein according to any one of [1] to [11], comprising:
   a step of, in a plant cell into which the polynucleotide according to [12] or the recombinant vector according to [13] has been introduced, or in a plant comprising the plant cell, expressing the polynucleotide.
[16] A method for producing human activin A, comprising the steps of:
   in a plant cell into which a polynucleotide encoding a recombinant activin A precursor protein according to [11] or a recombinant vector comprising the polynucleotide has been introduced, or in a plant comprising the plant cell, expressing the polynucleotide to generate the recombinant activin A precursor protein; and
   treating the recombinant activin A precursor protein with the protease.

In one embodiment, a recombinant activin A precursor protein having human activin A activity can be provided. "Human activin A activity" as used herein may mean the property of activating the Smad protein, which is a signaling factor, and/or the property of inducing iPS cells to differentiate into definitive endoderm.

In one embodiment, a novel method for producing human activin A can be provided. One aspect of this producing method may be simpler than the conventional methods. One aspect of this producing method may be more economical than the conventional methods. Moreover, one aspect of this producing method may have a higher production efficiency than the conventional methods.

The producing method of the present invention makes it possible to produce human activin A in the state of holding human activin A activity, unlike the producing methods utilizing microorganisms such as *Escherichia coli.*

### Brief Description of Drawings

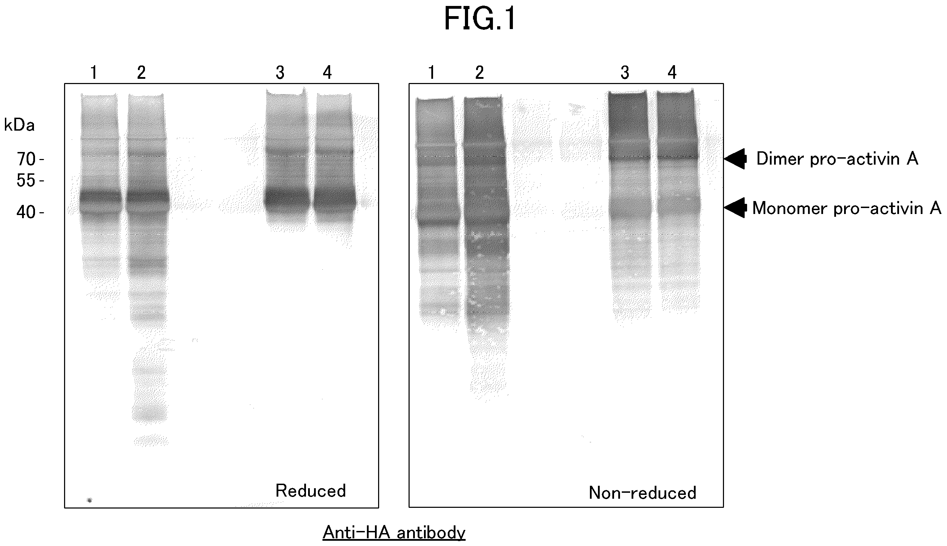

Fig. 1 illustrates photographs showing results of the western blotting with an anti-HA antibody. Lanes 1 and 2 show the results using extracts obtained by expressing a recombinant activin A precursor protein comprising a green sea turtle-derived prodomain in *Nicotiana benthamiana* and extracting proteins at the 4th day and the 5th day, respectively. Lanes 3 and 4 show the results using extracts obtained by expressing a recombinant activin A precursor protein comprising a zebrafish-derived prodomain in *Nicotiana benthamiana* and extracting proteins at the 4th day and the 5th day, respectively.

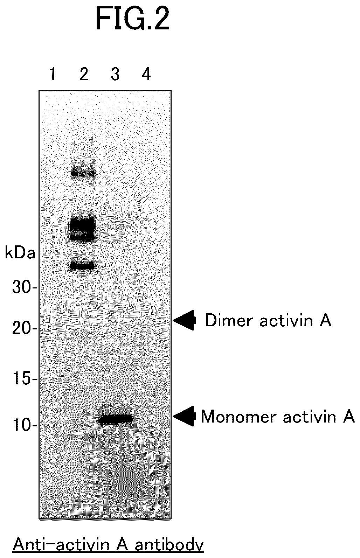

Fig. 2 illustrates a photograph showing results of the western blotting with an anti-activin A antibody. Lane 1 show a Co column-purified furinΔC protease ; lane 2 shows a recombinant activin A precursor protein comprising a Co column-purified zebrafish-derived prodomain; lane 3 shows reducing conditions for a mixture of the Co column-purified furinΔC protease and the recombinant activin A precursor protein comprising the zebrafish-derived prodomain; and lane 4 shows non-reducing conditions for the mixture of the Co column-purified furinΔC protease and the recombinant activin A precursor protein comprising the zebrafish-derived prodomain.

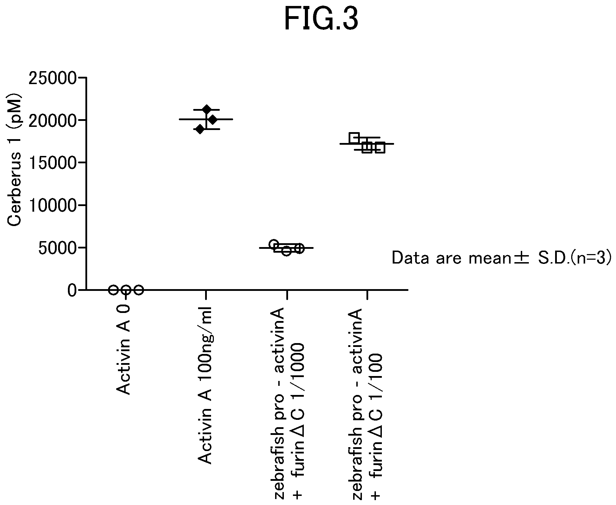

Fig. 3 illustrates a graph showing results of measuring the activin A bioactivity. The horizontal axis indicates, in order from the left, an experimental plot not containing activin A as a negative control (activin A 0); an experimental plot containing activin A produced by R&D systems as a positive control (activin A 100 ng/ml); an experimental plot containing activin A produced by the method of the present invention in an amount of 1/1000 of the medium (the recombinant activin A precursor protein comprising the zebrafish-derived prodomain + furinΔC protease 1/1000); and an experimental plot containing activin A produced by the method of the present invention in an amount of 1/100 of the medium (the recombinant activin A precursor protein comprising the zebrafish-derived prodomain + furinΔC protease 1/100). The vertical axis indicates the amount of Cerberus 1 (pM).

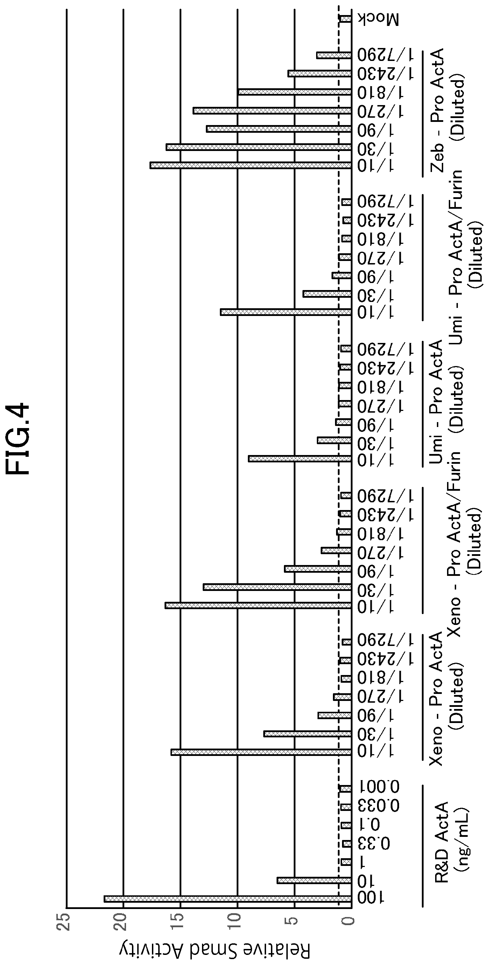

Fig. 4 illustrates a graph showing evaluation of activity of the recombinant activin A precursor protein by the Smad reporter assay. Fig. 4 shows, in order from the left, activin A produced by R&D systems (R&D ActA); a recombinant activin A precursor protein comprising an African clawed frog-derived prodomain (Xeno-Pro ActA); a recombinant activin A precursor protein comprising an African clawed frog-derived prodomain + furinΔC (Xeno-Pro ActA/Furin); a recombinant activin A precursor protein comprising a green sea turtle-derived prodomain (Umi-Pro ActA); a recombinant activin A precursor protein comprising a green sea turtle-derived prodomain + furinΔC (Umi-Pro ActA/Furin); a recombinant activin A precursor protein comprising a zebrafish-derived prodomain (Zeb-Pro ActA); and a sample to which no activin A was added (Mock). The horizontal axis in R&D ActA indicates the concentration (ng/mL), and the horizontal axis in those other than R&D ActA and Mock indicates the dilution ratio of the purified protein extract to the medium, or the dilution ratio of the partially purified mixed solution obtained by mixing the purified furinΔC and the recombinant activin A precursor protein to the medium. The vertical axis indicates a relative Smad activity.

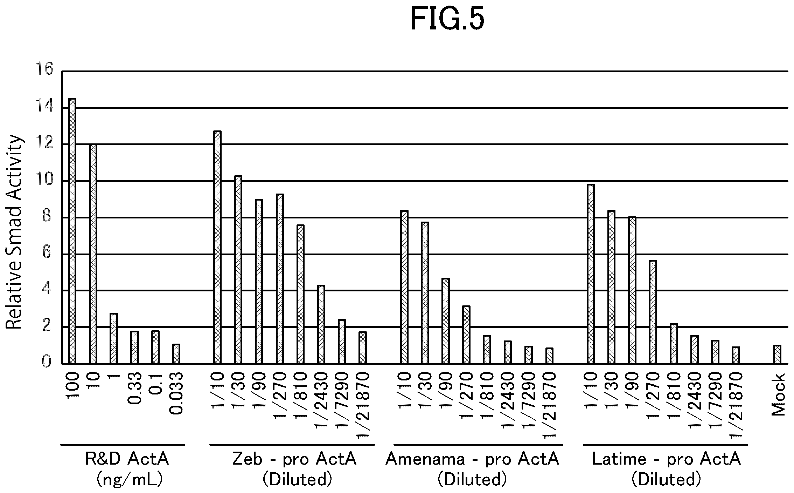

Fig. 5 illustrates a graph showing evaluation of activity of the recombinant activin A precursor protein by the Smad reporter assay. Fig. 5 shows, in order from the left, activin A produced by R&D systems (R&D ActA); a recombinant activin A precursor protein comprising a zebrafish-derived prodomain (Zeb-Pro ActA); a recombinant activin A precursor protein comprising a channel catfish-derived prodomain (Amenama-Pro ActA); a recombinant activin A precursor protein comprising a coelacanth-derived prodomain (Latime-Pro ActA); and a sample to which no activin A was added (Mock). The horizontal axis in R&D ActA indicates the concentration (ng/mL), and the horizontal axis in those other than R&D ActA and Mock indicates the dilution ratio of the purified protein extract to the medium. The vertical axis indicates a relative Smad activity.

### Description of Embodiments

### [Recombinant Activin A Precursor Protein]

An embodiment of the present invention relates to a recombinant activin A precursor protein (hereinafter, sometimes referred to as the "recombinant activin A precursor protein of the present invention") comprising: a human mature activin A sequence; and a non-human activin A prodomain, wherein the recombinant activin A precursor protein is not a naturally occurring activin A precursor protein. In one aspect of the present invention, the recombinant activin A precursor protein comprising: a human mature activin A sequence; and a non-human activin A prodomain may be used in a novel method for producing human activin A. In addition, it is known that the precursor protein of activin A comprising a non-human activin A prodomain has no human activin A activity; however, in one aspect of the present invention, it is possible that the recombinant activin A precursor protein comprising: a human mature activin A sequence; and a non-human activin A prodomain has human activin A activity.

"Human mature activin A sequence" as used herein means the C-terminus amino acid sequence of activin A which has been cleaved from the precursor protein of activin A (pro-activin A) expressed in a human living organism and which actually has activity,. The "human mature activin A sequence" is a sequence made of 116 amino acids and is an amino acid sequence at positions 304 to 419 in a sequence represented by SEQ ID NO: 8, which will be described later.

"Non-human activin A prodomain" as used herein means an inactive N-terminus amino acid sequence comprised in a precursor protein of activin A (pro-activin A) expressed in a living organism other than the human by the living organism, and is an amino acid sequence of a part excluding the C-terminus amino acid sequence of activin A which actually has activity and a protease recognition sequence adjacent to the N-terminus side of the C-terminus amino acid sequence.

In one embodiment of the present invention, the non-human activin A prodomain is an activin A prodomain of a mammal other than the human, a bird, an amphibian, a reptile, or a fish, and preferably an activin A prodomain of an amphibian, a reptile, or a fish. Although examples of the non-human activin A prodomain will be given below, the non-human activin A prodomain is not limited to only the examples given below, in the present invention.

In the present invention, the activin A prodomain of a fish preferably comprises a sequence having an amino acid sequence identity of 90% or more with an amino acid sequence of DTRRSGWHTL (SEQ ID NO: 1), and more preferably comprises a sequence having an amino acid sequence identity of 90% or more with the sequence represented by SEQ ID NO: 1 at positions corresponding to positions 187 to 196 of a sequence represented by SEQ ID NO: 2, which will be described later, but is not limited to these.

For example, the activin A prodomain of a fish includes those selected from the amino acid sequences of the activin A prodomains derived from the following living organisms: zebrafish (*Danio rerio*), channel catfish (*Ictalurus punctatus*), coelacanth (*Latimeria chalumnae*), *Sinocyclocheilus rhinocerous, Sinocyclocheilus anshuiensis, Sinocyclocheilus grahami,* common carp (*Cyprinus carpio*), goldfish (*Carassius auratus*), grass carp (*Ctenopharyngodon idellus*), *Pundamilia nyererei,* Mexican tetra (*Astyanax mexicanus*), *Maylandia zebra,* Japanese rice fish (*Oryzias latipes*), platy (*Xiphophorus maculatus*), guppy (*Poecilia reticulata*), sailfin molly (*Poecilia latipinna*), amazon molly (*Poecilia formosa*), Atlantic molly (*Poecilia mexicana*), barramundi *(Lates calcarifer),* Atlantic salmon (*Salmo salar*), northern pike (*Esox lucius*), Asian arowana *(Scleropages formosus), Maylandia Zebra,* haplochromis (*Haplochromis burutoni*), yellow croaker (*Larimichthys crocea*), mangrove killifish (*Kryptolebias marmoratus*), spiny chromis (*Acanthochromis polyacanthus*), yellowtail amberjack (*Seriola lalandi dorsalis*), spotted gar (*Lepisosteus oculatus*), silver salmon (*Oncorhynchus kisutch*), Atlantic herring (*Clupea harengus*), walking catfish (*Clarias batrachus*), piranha (*Pygocentrus nattereri*), rainbow trout (*Oncorhynchus mykiss*), ocellaris clownfish (*Amphiprion ocellaris*), bicolor damselfish (*Stegastes partitus*), greater amberjack (*Seriola dumerili*), Hong Kong catfish (*Clarias gariepinus*), tilapia *(Oreochromis niloticus*), annual killifish (*Austrofundulus limnaeus*), mummichog (*Fundulus heteroclitus*), southern platyfish (*Xiphophorus maculatus*), *Neolamprologus brichardi,* wrasse (*Labrus bergylta*), and Asian swamp eel (*Monopterus albus*).

The activin A prodomain of a fish may be selected from the amino acid sequences of the activin A prodomains derived from living organisms belonging to Cypriniformes, Siluriformes, or Coelacanthiformes. In addition, the activin A prodomain of a fish may be selected from the amino acid sequences of the activin A prodomains derived from living organisms belonging to Cyprinidae, Ictaluridae, or Latimeriidae, or may be selected from the amino acid sequences of the activin A prodomains derived from living organisms belonging to *Danio, Ictalurus* , or *Latimeria.*

In the present invention, the non-human activin A prodomain may be one having an amino acid sequence identity of 90% or more, more preferably 95% or more, and further preferably 98% or more with the amino acid sequence of the activin A prodomain of a fish not limited to the above-described examples.

In the present invention, the non-human activin A prodomain has an amino acid sequence identity of preferably 90% or more, more preferably 95% or more, and further preferably 98% or more with the amino acid sequence (SEQ ID NO: 2) of the activin A prodomain derived from zebrafish. In the present invention, the non-human activin A prodomain more preferably comprises the amino acid sequence of the activin A prodomain derived from zebrafish. In the present invention, the non-human activin A prodomain is further preferably made of the amino acid sequence of the activin A prodomain derived from zebrafish.

The amino acid sequence identity herein can be calculated using a publicly-known analysis tool, and for example, can be calculated using the identity algorithm BLAST (Basic local alignment search tool) of the National Center for Biotechnology Information (NCBI). In addition, for the calculation of the amino acid sequence identity, parameters of the default (initial setting) in the analysis tool may be used.

In the present invention, the non-human activin A prodomain has an amino acid sequence identity of preferably 90% or more, more preferably 95% or more, and further preferably 98% or more with the amino acid sequence (SEQ ID NO: 3) of the activin A prodomain derived from channel catfish. In the present invention, the non-human activin A prodomain more preferably comprises the amino acid sequence of the activin A prodomain derived from channel catfish. In the present invention, the non-human activin A prodomain is further preferably made of the amino acid sequence of the activin A prodomain derived from channel catfish.

In the present invention, the non-human activin A prodomain has an amino acid sequence identity of preferably 90% or more, more preferably 95% or more, and further preferably 98% or more with the amino acid sequence (SEQ ID NO: 4) of the activin A prodomain derived from coelacanth. In the present invention, the non-human activin A prodomain more preferably comprises the amino acid sequence of the activin A prodomain derived from coelacanth. In the present invention, the non-human activin A prodomain is further preferably made of the amino acid sequence of the activin A prodomain derived from coelacanth.

For example , the activin A prodomain of an amphibian includes those selected from the amino acid sequences of the activin A prodomains derived from the following living organism: African clawed frog (*Xenopus laevis*), Japanese fire belly newt (*Cynops pyrrhogaster*), western clawed frog (*Xenopus tropicalis*), *Nanorana parkeri,* common coqui (*Eleutherodactylus coqui*), and American bullfrog (*Lithobates catesbeiana*).

The activin A prodomain of an amphibian may be selected from the amino acid sequences of the activin A prodomains derived from living organisms belonging to Anura. In addition, the activin A prodomain of an amphibian may be selected from amino acid sequences of the activin A prodomains derived from living organisms belonging to Pipidae, or may be selected from amino acid sequences of the activin A prodomains derived from living organisms belonging to *Xenopus.*

In the present invention, the non-human activin A prodomain may be one having an amino acid sequence identity of 90% or more, more preferably 95% or more, and further preferably 98% or more with the amino acid sequence of the activin A prodomain of an amphibian not limited to the above-described examples.

In the present invention, the non-human activin A prodomain has an amino acid sequence identity of preferably 90% or more, more preferably 95% or more, and further preferably 98% or more with the amino acid sequence (SEQ ID NO: 5) of the activin A prodomain derived from African clawed frog. In the present invention, the non-human activin A prodomain more preferably comprises the amino acid sequence of the activin A prodomain derived from African clawed frog. In the present invention, the non-human activin A prodomain is further preferably made of the amino acid sequence of the activin A prodomain derived from African clawed frog.

For example , the activin A prodomain of a reptile includes those selected from amino acid sequence of activin A prodomain derived from the following living organisms: green sea turtle (*Chelonia mydas*), Indian python (*Python molurus*), green anole (*Anolis carolinensis*), central bearded dragon (*Pogona vitticeps*), Japanese gecko (*Gekko japonicus*), king cobra (*Ophiophagus hannah*), garter snake (*Thamnophis sirtalis*), brown spotted pit viper (*Protobothrops mucrosquamatus*), painted turtle (*Chrysemys picta*), Chinese alligator (*Alligator sinensis*), Indian gavial (*Gavialis gangeticus*), and American alligator (*Alligator mississippiensis*).

The activin A prodomain of a reptile may be selected from the amino acid sequences of the activin A prodomains derived from living organisms belonging to Testudines. In addition, the activin A prodomain of a reptile may be selected from the amino acid sequences of the activin A prodomains derived from living organisms belonging to Cheloniidae, or may be selected from the amino acid sequences of the activin A prodomains derived from living organisms belonging to *Chelonia.*

In the present invention, the non-human activin A prodomain may be one having an amino acid sequence identity of 90% or more, more preferably 95% or more, and further preferably 98% or more with the amino acid sequence of the activin A prodomain of a reptile not limited to the above-described examples.

In the present invention, the non-human activin A prodomain has an amino acid sequence identity of preferably 90% or more, more preferably 95% or more, and further preferably 98% or more with the amino acid sequence (SEQ ID NO: 6) of the activin A prodomain derived from green sea turtle. In the present invention, the non-human activin A prodomain more preferably comprises the amino acid sequence of the activin A prodomain derived from green sea turtle. In the present invention, the non-human activin A prodomain is further preferably made of the amino acid sequence of the activin A prodomain derived from green sea turtle.

"Not a naturally occurring activin A precursor protein" as used herein means that the recombinant activin A precursor protein is different in amino acid sequence from the precursor proteins of activin A (pro-activin A) produced by the expression of genes of natural living organisms whose genes are not modified. In other words, the recombinant activin A precursor protein comprising: a human mature activin A sequence; and a non-human activin A prodomain of the present invention is different in amino acid sequence from the precursor proteins of activin A produced by the expression of genes of natural living organisms whose genes are not modified.

For example, some mature activin A sequences in primates such as monkeys have the same sequence as the mature activin A sequence of the human. Hence, a activin A precursor protein comprising: the mature activin A sequence of a monkey; and the activin A prodomain of the monkey is deemed to be the same as the activin A precursor protein comprising: the mature activin A sequence of the human and the activin A prodomain of the monkey, and "not a naturally occurring activin A precursor protein" as used herein is intended to mean that such an activin A precursor protein is excluded from the scope of the present invention.

In the recombinant activin A precursor protein comprising: a human mature activin A sequence; and a non-human activin A prodomain of the present invention, the human mature activin A sequence is preferably located closer to the C-terminus than the non-human activin A prodomain. In addition, the recombinant activin A precursor protein of the present invention more preferably comprises a sequence specifically cleavable by a protease between the human mature activin A sequence and the non-human activin A prodomain. The sequence specifically cleavable by a protease is preferably adjacent to the human mature activin A sequence and the non-human activin A prodomain and is more preferably adjacent to the C-terminus side of the non-human activin A prodomain and to the N-terminus side of the human mature activin A sequence.

The sequence specifically cleavable by a protease includes a furin recognition sequence, a TEV protease recognition sequence, an HRV 3C protease recognition sequence, a thrombin recognition sequence, a factor Xa recognition sequence, an enterokinase recognition sequence, and the like, but the sequence specifically cleavable by a protease is not limited to these. The sequence specifically cleavable by a protease is preferably one that can be cleaved on the C-terminus side of the sequence, and is more preferably a furin recognition sequence. The furin recognition sequence is a sequence that generally has arginine at the C-terminus (has lysine at the C-terminus in some cases), and in which the C-terminus side can be cleaved by furin. For example, a standard furin recognition sequence can be expressed by an amino acid sequence represented by R-X-(K/R)-R (X is any amino acid), but the furin recognition sequence is not limited to this.

The recombinant activin A precursor protein of the present invention may comprise a desired tag bound in a genetically engineered manner, for example, the His tag, the HA tag, the FLAG tag, the Myc tag, or the like. As the tag, a tag that does not affect the conformation of the activin A precursor protein is preferably used, and a tag formed of 5 to 15 amino-acid residues is more preferable. In the case where the recombinant activin A precursor protein of the present invention comprises a tag, the tag is preferably located on the N-terminus side of the non-human activin A prodomain.

### [Polynucleotide That Encodes Recombinant Pro-activin A Protein]

One embodiment of the present invention relates to a polynucleotide encoding the recombinant activin A precursor protein of the present invention. The polynucleotide can be synthesized by those skilled in the art based on the technical common knowledge.

The polynucleotide encoding the recombinant activin A precursor protein of the present invention may comprise a nucleic acid sequence encoding any signal sequence bounded to the N-terminus side in a genetically engineered manner. The signal sequence is preferably an endoplasmic reticulum insertion signal sequence, and an endoplasmic reticulum insertion signal sequence known by those skilled in the art such as one derived from thale cress may be used.

In the case where the recombinant activin A precursor protein of the present invention comprises a tag on the N-terminus side of the non-human activin A prodomain, the polynucleotide encoding the recombinant activin A precursor protein preferably comprises a nucleic acid sequence encoding the signal sequence the side closer to the N-terminus than the nucleic acid sequence encoding the tag.

### [Recombinant Vector]

One embodiment of the present invention relates to a recombinant vector comprising: a polynucleotide encoding the recombinant activin A precursor protein of the present invention. The usable vector may be one that can functionally insert the polynucleotide and can carry the different nucleic acid fragment into a host cell to allow the fragment to be replicated or expressed in the host cell, and includes, for example, plasmids, phages, cosmids, and viruses. The vector can be selected by those skilled in the art as appropriate depending on the type of an assumed host cell.

In the present invention, the host cell includes, for example, plant cells, insect cells, fungus cells (yeast), prokaryotic cells (*Escherichia coli*), animal cells (CHO). Among these, since plant cells are assumed as the host cell, the vector is preferably an expression vector for plant cells, such as pRI 201 DNA series (TAKARA BIO Inc.), but is not limited to these. An appropriate vector may be selected and used depending on the type of a plant cell.

### [Transformant]

One embodiment of the present invention relates to a transformant comprising: a polynucleotide encoding the recombinant activin A precursor protein of the present invention, or a transformant comprising: a recombinant vector comprising a polynucleotide encoding the recombinant activin A precursor protein of the present invention.

"Transformant" as used herein means a host or host cell in which a different nucleic acid fragment has been introduced. The nucleic acid fragment for transformation, that is, the polynucleotide encoding the recombinant activin A precursor protein of the present invention may be incorporated in the genome of the host or host cell or may not be incorporated. For example, the nucleic acid fragment for transformation may be maintained in an episome or a plasmid. The introduction of a gene may be conducted by those skilled in the art as appropriate based on the technical common knowledge. For example, the transformant is preferably a plant cell or a plant, and as a method for introducing a gene into a plant cell or a plant, the agroinfiltration method, the plant virus vector method, and the like are known.

### [Method for Producing Recombinant Pro-activin A Protein]

One embodiment of the present invention is a method for producing the recombinant activin A precursor protein of the present invention. The method for producing a recombinant activin A precursor protein of the present invention comprises a step of, in a plant cell into which the polynucleotide encoding the recombinant activin A precursor protein of the present invention or a recombinant vector comprising the polynucleotide has been introduced, or in a plant comprising the plant cell, expressing a polynucleotide. This allows the plant cell or the plant to produce the target recombinant activin A precursor protein.

In the method for producing a recombinant activin A precursor protein of the present invention, the expression of the polynucleotide may be transient or stable in a plant cell or a plant. The culture conditions for the plant cell may be selected by those skilled in the art as appropriate based on the technical common knowledge in accordance with the type of the plant cell used. For example, the culture medium and the conditions such as the temperature, the humidity, and the concentration of carbon dioxide, and the like of the environment where the culture is to be conducted are not particularly limited as long as the culture medium and the conditions are suitable for each cell, and may be selected from a conventionally known culture medium and conditions. The cultivating conditions for a plant can also be selected by those skilled in the art as appropriate based on the technical common knowledge in accordance with the type of the plant used.

The plant used for the method for producing a recombinant activin A precursor protein of the present invention is not particularly limited, but preferably is a plant that can be infected with agrobacterium. For example, the plant may be a dicot or may be a monocot. The dicot includes Solanaceae plants such as tobacco, tomato, and potato, Brassicaceae plants such as thale cress, rapeseed, broccoli, and cabbage, Asteraceae plants such as crown daisy and lettuce, Rosaceae plants such as strawberry, Japanese apricot, apple, and peach, Fabaceae plants such as soybean, peanut, and mung bean, Apiaceae plants such as carrot and celery, Cucurbitaceae plants such as melon, watermelon, and cucumber, Convolvulaceae plants such as Japanese morning glory and sweet potato, Chenopodiaceae plants such as spinach, quinoa, and sugar beet, and the like. The monocot includes Poaceae plants such as rice, wheat, barley, and corn, Liliaceae plants such as garlic, onion, and Welsh onion, and the like.

The plant used for the method for producing a recombinant activin A precursor protein of the present invention is preferably a Solanaceae plant, and more preferably tobacco. Tobacco includes, for example, *Nicotiana benthamiana, Nicotiana tabacum, Nicotiana rustica, Nicotiana tomentosiformis, Nicotiana sylvestris,* and the like, and is preferably *Nicotiana benthamiana.*

The plant cell used for the method for producing a recombinant activin A precursor protein of the present invention is also not particularly limited, and cells of the above-described plants may be used.

In the method for producing a recombinant activin A precursor protein of the present invention, in the case where the recombinant activin A precursor protein comprises a sequence specifically cleavable by a protease between the human mature activin A sequence and the non-human activin A prodomain, the sequence specifically cleavable by a protease is one that is not cleaved by a protease present inside the plant cell to be used as the host cell.

The method for producing a recombinant activin A precursor protein of the present invention may further comprise a step of extracting, from the plant cell or the plant, a recombinant activin A precursor protein generated in the plant cell or the plant. For example, the protein can be extracted from the plant cell by breaking the tissue using something like a mixer or a mortar with water or a buffer. Such an extracting step can be conducted by those skilled in the art as appropriate based on the technical common knowledge.

The method for producing a recombinant activin A precursor protein of the present invention may further comprise a step of purifying the extract containing the recombinant activin A precursor protein extracted from the plant cell or the plant. For example, it is possible to add an epitope tag to the recombinant protein in advance, and purify the recombinant protein by a purifying method according to the added epitope. The epitope tag includes the histidine tag, the HA tag, the GST tag, the MBP tag, the MYC tag, the DYKDDDDK tag, the streptavidin tag, and the like. Such a purifying step can be conducted by those skilled in the art as appropriate based on the technical common knowledge.

### [Method for Producing Human Activin A]

One embodiment of the present invention is a method for producing human activin A (hereinafter, sometimes referred to as the "method for producing human activin A of the present invention"), and human activin A produced by this producing method may have human activin A activity.

The method for producing human activin A of the present invention comprises at least the following two steps:
1. in a plant cell into which a polynucleotide encoding the recombinant activin A precursor protein of the present invention or a recombinant vector comprising the polynucleotide has been introduced, or in a plant comprising the plant cell, expressing the polynucleotide to generate the recombinant activin A precursor protein. Here, the recombinant activin A precursor protein of the present invention comprises a human mature activin A sequence and a non-human activin A prodomain and also comprises a sequence specifically cleavable by a protease between the human mature activin A sequence and the non-human activin A prodomain; and
2. treating the recombinant activin A precursor protein with the protease.

In the method for producing human activin A of the present invention, the recombinant activin A precursor protein is of an aspect that comprises a human mature activin A sequence and a non-human activin A prodomain and also comprises a sequence specifically cleavable by a protease between the human mature activin A sequence and the non-human activin A prodomain, among the above-described recombinant activin A precursor protein of the present invention. The sequence specifically cleavable by a protease is preferably adjacent to the human mature activin A sequence and the non-human activin A prodomain, and is more preferably adjacent to the C-terminus side of the non-human activin A prodomain and the N-terminus side of the human mature activin A sequence.

The sequence specifically cleavable by a protease includes a furin recognition sequence, a TEV protease recognition sequence, an HRV 3C protease recognition sequence, a thrombin recognition sequence, a factor Xa recognition sequence, an enterokinase recognition sequence, and the like, but the sequence specifically cleavable by a protease is not limited to these. The sequence specifically cleavable by a protease is preferably one that can be cleaved on the C-terminus side of the sequence, and is more preferably a furin recognition sequence. The furin recognition sequence is a sequence that generally has arginine at the C-terminus (has lysine at the C-terminus in some cases), and in which the C-terminus side can be cleaved by furin. For example, a standard furin recognition sequence can be expressed by an amino acid sequence represented by R-X-(K/R)-R (X is any amino acid), but the furin recognition sequence is not limited to this.

In the method for producing human activin A of the present invention, the expression of the polynucleotide may be transient or stable in a plant cell or a plant. The culture conditions for the plant cell may be selected by those skilled in the art as appropriate based on the technical common knowledge in accordance with the type of the plant cell used. For example, the culture medium and the conditions such as the temperature, the humidity, and the concentration of carbon dioxide, and the like of the environment where the culture is to be conducted are not particularly limited as long as the culture medium and the conditions are suitable for each cell, and may be selected from a conventionally known culture medium and conditions. The cultivating conditions for a plant can also be selected by those skilled in the art as appropriate based on the technical common knowledge in accordance with the type of the plant used.

The plant used for the method for producing human activin A of the present invention is not particularly limited, but preferably is a plant that can be infected with agrobacterium. For example, the plant may be a dicot or may be a monocot. The dicot includes Solanaceae plants such as tobacco, tomato, and potato, Brassicaceae plants such as thale cress, rapeseed, broccoli, and cabbage, Asteraceae plants such as crown daisy and lettuce, Rosaceae plants such as strawberry, Japanese apricot, apple, and peach, Fabaceae plants such as soybean, peanut, and mung bean, Apiaceae plants such as carrot and celery, Cucurbitaceae plants such as melon, watermelon, and cucumber, Convolvulaceae plants such as Japanese morning glory and sweet potato, Chenopodiaceae plants such as spinach, quinoa, and sugar beet, and the like. The monocot includes Poaceae plants such as rice, wheat, barley, and corn, Liliaceae plants such as garlic, onion, and Welsh onion, and the like.

The plant used for the method for producing a recombinant activin A precursor protein of the present invention is preferably a Solanaceae plant, and more preferably tobacco. Tobacco includes, for example, *Nicotiana benthamiana, Nicotiana tabacum, Nicotiana rustica, Nicotiana tomentosiformis, Nicotiana sylvestris,* and the like, and is preferably *Nicotiana benthamiana.*

The plant cell used for the method for producing a recombinant activin A precursor protein of the present invention is also not particularly limited, and cells of the above-described plants may be used.

In the method for producing human activin A of the present invention, a human mature activin A sequence is cleaved by treating the recombinant activin A precursor protein generated in the plant cell or in the plant with the protease. The human mature activin A sequence itself forms human activin A, and human activin A produced by the method for producing human activin A of the present invention in this manner can be in the state of holding the activity.

The protease that can be used in the method for producing human activin A of the present invention includes the furin protease, the TEV protease, the HRV 3C protease, the thrombin, the factor Xa, the enterokinase, and the like, but is not limited to these. The protease is preferably one that cleaves the C-terminus of the recognition sequence, and is more preferably the furin protease. Note that although the furin protease is of lipid-raft type and naturally an insoluble protease, there is a report that the furin protease was converted into secreted soluble type by deleting the lipid-raft region using an insect cell (Bravo, D. a., Gleason, J. B., Sanchez, R. I., Roth, R. a. & Fuller, R. S. Accurate and efficient cleavage of the human insulin proreceptor by the human proprotein-processing protease furin. Characterization and kinetic parameters using the purified, secreted soluble protease expressed by a recombinant baculovirus. J. Biol. Chem. 269, 25830-25837 (1994), the contents of which are incorporated herein by reference), and it is possible to use a furin protease obtained by deleting the lipid-raft region present at the C-terminus of such furin protease in the method for producing human activin A of the present invention.

The treatment with the protease may be conducted in a plant cell or a plant in which the recombinant activin A precursor protein has been generated. In this case, the protease may be a protease that is spontaneously present in a plant cell or in a plant, or a protease that is not spontaneously present in a plant cell or in a plant may be caused to express in a plant cell or in a plant by transformation using gene transfer. Such transfer of a gene into a plant cell or a plant can be conducted by those skilled in the art as appropriate based on the technical common knowledge, and can be conducted by a method such as the agroinfiltration method or the plant virus vector method.

In addition, the treatment with the protease may be conducted outside a plant cell or a plant in which the recombinant activin A precursor protein has been generated. In this case, the protease may be a commercially-available protease or a protease prepared separately from the recombinant activin A precursor protein. For example, a furin protease obtained by deleting the lipid-raft region present at the C-terminus of a furin protease is one converted into secreted soluble type, and it is possible to react the furin protease with the recombinant activin A precursor protein outside a plant cell or a plant by preparing this furin protease separately from the recombinant activin A precursor protein and using the furin protease.

The method for producing human activin A of the present invention may further comprise a step of extracting, from the plant cell or the plant, a recombinant activin A precursor protein generated in the plant cell or the plant. For example, the protein can be extracted from the plant cell by breaking the tissue using something like a mixer or a mortar with water or a buffer. Such an extracting step can be conducted by those skilled in the art as appropriate based on the technical common knowledge. This extracting step is conducted before the step of treating the recombinant activin A precursor protein with the protease.

The method for producing human activin A of the present invention may further comprise a step of purifying the extract containing the recombinant activin A precursor protein extracted from the plant cell or the plant. For example, it is possible to add an epitope tag to the recombinant protein in advance, and purify the recombinant protein by a purifying method according to the added epitope. The epitope tag includes the histidine tag, the HA tag, the GST tag, the MBP tag, the MYC tag, the DYKDDDDK tag, the streptavidin tag, and the like. Such a purifying step can be conducted by those skilled in the art as appropriate based on the technical common knowledge. This purifying step is conducted before the step of treating the recombinant activin A precursor protein with the protease.

In the case where the step of treating the recombinant activin A precursor protein with the protease has been conducted in a plant cell or in a plant, the method for producing human activin A of the present invention may further comprise a step of extracting, from the plant cell or the plant, human activin A generated in the plant cell or the plant after the treating step. For example, the protein can be extracted from the plant cell by breaking the tissue using something like a mixer or a mortar with water or a buffer. Such an extracting step can be conducted by those skilled in the art as appropriate based on the technical common knowledge.

The method for producing human activin A of the present invention may further comprise a step of purifying the extract containing the human activin A extracted from the plant cell or the plant. For example, it is possible to add an epitope tag to the recombinant protein in advance, and purify the recombinant protein by a purifying method according to the added epitope. The epitope tag includes the histidine tag, the HA tag, the GST tag, the MBP tag, the MYC tag, the DYKDDDDK tag, the streptavidin tag, and the like. Such a purifying step can be conducted by those skilled in the art as appropriate based on the technical common knowledge.

### [Examples]

Hereinafter, the present invention will be described in further detail with reference to specific Examples. However, the scope of the present invention is not limited to these Examples.

### [Materials and Method]

### 1. Used Plant and its Cultivation Method

As the plant, tobacco (cultivar: *Nicotiana benthamiana*) was used. A cultivation soil was prepared by mixing Kumiai Nippi Engei Baido No. 1 and vermiculite in 1:1, and the cultivation soil thus prepared was put into a plug tray. Tobacco seeds were sowed on the plug tray, were cultivated for about 4 weeks, and were then transplanted into a No. 3 vinyl pot. For agroinfiltration, the plant in the second to third week after the transplantation in the vinyl pot was used. The cultivation was conducted at a light intensity of about 70 µmol m⁻² s⁻¹ before the agroinfiltration (in the lower stage of the Biotron B room), and at a light intensity of about 150 µmol m⁻² s⁻¹ after the agroinfiltration (in the upper stage of the Biotron B room).

### 2. Construction of Plasmid Vector and Artificial Gene Synthesis

Various insert DNAs, which were artificial genes, were inserted between the NdeI site immediately below the AtADH 5' UTR and the SalI site further downstream of the AtADH 5' UTR using the pRI201-AN vector (TAKARA BIO Inc.). The Gibson Assembly (Registered Trademark) Master Mix was used for the insertion of the insert DNAs.

The artificial genes were synthesized utilizing the GeneArt (Registered Trademark) StringsTM DNA Fragments service of Thermo Fisher Scientific. The synthesized genes were optimized in conformity with the frequency of codon occurrence of *Nicotiana benthamiana* using the GeneArt (Registered Trademark) GeneOptimizer (Registered Trademark) provided by Thermo Fisher Scientific.

The artificial genes actually used are shown in Table 1.

**[Table 1]**

| Description of Sequence | SEQ ID NO: |
|---|---|
| (ABC-SP)-(His tag)-(HA tag)-(zebrafish Pro-domain) -(Furin)-(human activin A) | SEQ ID NOs: 7, 8 |
| (ABC-SP)-(His tag)-(HA tag)-(channel catfish Pro-domain) -(Furin)-(human activin A) | SEQ ID NOs: 9, 10 |
| (ABC-SP)-(His tag)-(HA tag)-(coelacanth Pro-domain) -(Furin)-(human activin A) | SEQ ID NOs: 11, 12 |
| (ABC-SP)-(His tag)-(HA tag)-(African clawed frog Pro-domain) -(Furin)-(human activin A) | SEQ ID NOs: 13, 14 |
| (ABC-SP)-(His tag)-(green sea turtle Pro-domain) -(Furin)-(human activin A) | SEQ ID NOs: 15, 16 |

ABC-SP: endoplasmic reticulum insertion signal peptide derived from Arabidopsis basic chitinase
Zebrafish Pro-domain: the activin A prodomain derived from zebrafish
Channel catfish Pro-domain: the activin A prodomain derived from channel catfish Coelacanth Pro-domain: the activin A prodomain derived from coelacanth
African clawed frog Pro-domain: the activin A prodomain derived from African clawed frog
Green sea turtle Pro-domain: the activin A prodomain derived from green sea turtle Furin: the furin recognition sequence
Human activin A: the human mature activin A sequence

### 3. Line and Transformation of Escherichia coli and Agrobacterium

The transformation was conducted using NEB (Registered Trademark) 10-beta strain (NEB) as *Escherichia coli* and LBA4404 strain (TAKARA BIO Inc.) as agrobacterium in accordance with the protocols attached to the products. First, each plasmid vector was increased in *Escherichia coli* and was corrected, and subsequently each plasmid vector was introduced into agrobacterium. For the culture of both *Escherichia coli* and agrobacterium, an LB medium was used. *Escherichia coli* was cultured at 37°C and agrobacterium was cultured at 28°C.

### 4. Agroinfiltration

Agrobacterium holding each transgene was precultured in 3 ml of the LB medium at 28°C overnight. The agrobacterium suspension thus precultured was 10-fold diluted in the LB medium (1 ml of the agrosolution per 9 ml of the LB), and acetosyringone was added such that the final concentration became 100 µM, followed by main culture for 4 hours. The suspension subjected to the main culture was centrifuged at 4000 g for 5 minutes to collect bacterial cells. The pellets thus collected were suspended such that O.D.600 = 0.5 in a 10 mM MES (pH 5.5) + 10 mM MgCl₂ solution, and acetosyringone was added such that the final concentration became 200 µM. The bacterial culture prepared in this manner was allowed to stand at room temperature 25°C for 1 hour to 3 hours. Thereafter, a 1 ml needleless syringe was used. The syringe was brought into contact with the lower side of the tobacco leaf, then the upper side was pinched with fingers to cause the pressurized state, and the bacterial culture was injected into the leaf (agroinfiltration). After the agroinfiltration, cultivation was conducted at about 150 µmol m⁻² s⁻¹ (in the upper stage of the Biotron B room), and sampling was conducted between the 4th day and the 7th day after the inoculation.

### 5. Extraction of Protein

After the leaf subjected to the agroinfiltration was collected and the fresh weight was measured, a refrigerate extraction buffer was added in an amount 3 times the fresh weight, and the leaf in the raw or frozen at -80°C was pulverized with a mortar. As the extraction buffer, a buffer obtained by adding NaCl to D-PBS such that the final concentration became 0.3 to 1 M, further adding Tween-20 in 0.5% to 1%, and adjusting pH to 7.4 was used. For use in metal affinity purification, imidazole was added such that the final concentration became 5 to 15 mM. In addition, commercially-available xTractor buffer (TAKARA BIO Inc.) was also used as a buffer dedicated for the metal affinity purification.

### 6. Metal Affinity Purification

The protein extract was centrifuged with CR22G III centrifuge (Hitachi, Ltd.) at 18,000 g at 4°C for 15 minutes to cause fragments of cells to precipitate, and the supernatant was recovered. The supernatant was transferred into himac 50 ml culture tube (Hitach, Ltd.) and centrifuged at 32,200 g at 4°C for 30 to 60 minutes to cause finer fragments to precipitate, and the supernatant was recovered. An appropriate amount of TALON (Registered Trademark) Metal Affinity Resin (TAKARA BIO Inc.) or His60 Ni Superflow Resin (TAKARA BIO Inc.) was added to the supernatant, followed by shaking at 4°C for 20 minutes or more to allow the Resin to adsorb the protein. Thereafter, the solution containing the Resin was transferred into a gravity column to recover the flow-through. Next, washing was conducted with a Washbuffer (D-PBS + final concentration 0.3 M NaCl, 15 mM imidazole, pH 7.4) in an amount 5 times the volume of the Resin. The elution was conducted by adding an elution buffer for Co resin (D-PBS + final concentration 0.3 M NaCl, 150 mM imidazole, 1 mM CaCl₂, pH 7.4) or an elution buffer for Ni resin (D-PBS + final concentration 0.3 M NaCl, 0.5 M imidazole, 1 mM CaCl₂, pH 8.0) in an amount twice the volume of the Resin to recover the eluate.

### 7. Western Blotting

The sample at the time of protein electrophoresis was heated at 100°C for 3 minutes after a sample buffer solution (for SDS-PAGE, 6-time concentrated, containing a reducing agent) pH 6.0 (Nacalai Tesque, Inc.) was added. DTT or mercaptoethanol was used as a reducing agent. The sample for non-reduction was prepared by adding NuPAGE (Registered Trademark) 4X LDS Sample Buffer and treating at 70°C for 10 minutes. As the polyacrylamide gel, e·PAGEL 15% (ATTO) was used to conduct electrophoresis. Thereafter, semi-dry blotting device PoweredBlot Ace (ATTO) was used to transfer the protein to the PVDF membrane. An antibody to the target protein was used as the primary antibody and an HRP antibody to each host animal was used as the secondary antibody. As the anti-activin antibody, a goat-derived polyclonal antibody (R&D systems, product code AF338) was used. As the anti-HA antibody, a rat monoclonal antibody Anti-HA High Affinity (Roche) was used. Detection was conducted by LAS-3000(Fujifilm) using ECLTM Prime Western Blotting Detection Reagent of GE Healthcare.

The results of detection with the anti-HA antibody are illustrated in Fig. 1. Lanes 1 and 2 show the results using extracts obtained by expressing a recombinant activin A precursor protein comprising a green sea turtle-derived prodomain in *Nicotiana benthamiana* and extracting proteins at the 4th day and the 5th day, respectively. Lanes 3 and 4 show the results using extracts obtained by expressing a recombinant activin A precursor protein comprising a zebrafish-derived prodomain in *Nicotiana benthamiana* and extracting proteins at the 4th day and the 5th day, respectively.

As shown in Fig. 1, a band was detected near an estimated molecular weight (about 44 kDa) of a monomer pro-activin A in the reduced state and a band was detected near an estimated molecular weight (about 88 kDa) of a dimer pro-activin A in the non-reduced state. These suggested that the produced proteins were present in the state where the disulfide bond was able to be cleaved by the reducing agent and were folded in a state similar to the natural state.

The results of detection with the anti-activin A antibody are shown in Fig. 2. Lane 1 shows only the result obtained by deleting the lipid-raft region present at the C-terminus of the furin protease and adding an His tag to synthesize a soluble furin protease (furinΔC protease), followed by the Co column purification. Lane 2 shows only a recombinant activin A precursor protein comprising a Co column-purified zebrafish-derived prodomain. Lane 3 shows the reducing conditions for a mixture of the Co column-purified furinΔC protease and the recombinant activin A precursor protein comprising the zebrafish-derived prodomain. Lane 4 shows the non-reducing conditions for the mixture of the Co column-purified furinΔC protease and the recombinant activin A precursor protein comprising the zebrafish-derived prodomain. The furinΔC protease was obtained, using a plant cell, by synthesizing a soluble furin protease formed by deleting a lipid-raft region present at the C-terminus of a furin protease to convert the furin protease into the secreted soluble type while adding an His tag, as described in Bravo, D. a., Gleason, J. B., Sanchez, R. I., Roth, R. a. & Fuller, R. S. Accurate and efficient cleavage of the human insulin proreceptor by the human proprotein-processing protease furin. Characterization and kinetic parameters using the purified, secreted soluble protease expressed by a recombinant baculovirus. J. Biol. Chem. 269, 25830-25837 (1994) (Although the above-described literature uses an insect cell, the synthesis can be made in the same manner using a plant cell). The furinΔC protease was synthesized and purified separately from the recombinant activin A precursor protein comprising the zebrafish-derived prodomain. In this way, the possibility that as a result of separation of a prodomain and a mature sequence, which is an active domain, due to the cleavage of the recombinant activin A precursor protein comprising the zebrafish-derived prodomain by the furin protease, the mature sequence was decomposed by an endogenous protease in the plant was avoided.

The furinΔC protease and the recombinant activin A precursor protein comprising the zebrafish-derived prodomain, which were purified separately, were mixed in a concentration ratio of 7:5 and treated at 30°C for 30 minutes. As a result, most of the prodomains were cleaved and a band, which was considered to be active under the reducing conditions, was detected near the estimated molecular weight (about 12 kDa). In addition, under the non-reducing conditions, a thin band was detected near the estimated molecular weight (about 24 kDa) of a dimer, because of the poor recognition of the antibody, or other reasons.

### 8. Measurement of Bioactivity of Activin A

Since the band was detected near about 12 kDa, which was the estimated molecular weight of the active human activin A by cleaving the recombinant activin A precursor protein comprising the zebrafish-derived prodomain with the furinΔC protease, the bioactivity of the activin A based on the differentiation from the iPS cells to the definitive endoderm (DE) as an index was confirmed as follows.

Human iPS cells (1231A3 strain) maintained and cultured in an AK01 medium were sowed into a 96 well plate coated with iMatrix at 4 x 104 cells/well. After 24 hours, the medium was replaced with a DE differentiation medium (the activin A produced by the method of the present invention or a commercial product, 2% B27 supplement, 3 µM CHIR99021) to initiate differentiation induction. The next day, the medium was replaced with a medium having the same composition and excluding CHIR99021, and thereafter, the medium was replaced every day. The evaluation was made using a commercial product of R&D systems as control, and the amount of Cerberus 1 in the culture supernatant was measured as an index of the DE differentiation. Note that Cerberus 1 is a secretory protein that expresses specifically to the DE stage, and activin A is known to be essential for the differentiation from iPS cells to DE.

Activin A produced by the method of the present invention was evaluated using a partially purified mixed solution obtained by mixing furinΔC purified through the Co column and the recombinant activin A precursor protein comprising the zebrafish-derived prodomain. Since the recombinant activin A precursor protein synthesized in a plant by the method of the present invention and purified through the Co column was in a partially purified state and the concentration was unclear, the partially purified mixed solution was added each in an amount of 1/1000 or 1/100 of the medium and used. The supernatant was recovered at the 5th day after the differentiation induction, and the amount of Cerberus 1 in the culture supernatant was quantified by the ELISA method using ES/iPS Differentiation Monitoring Kit (Dojindo, #ES01).

The results of measuring the activin A bioactivity are shown in Fig. 3. The horizontal axis indicates, in order from the left, an experimental plot not containing activin A as a negative control (activin A 0); an experimental plot containing activin A produced by R&D systems as a positive control (activin A 100 ng/ml); an experimental plot containing activin A produced by the method of the present invention in an amount of 1/1000 of the medium (the recombinant activin A precursor protein comprising the zebrafish-derived prodomain + furinΔC 1/1000); and an experimental plot containing activin A produced by the method of the present invention in an amount of 1/100 of the medium (the recombinant activin A precursor protein comprising the zebrafish-derived prodomain + furinΔC 1/100). The vertical axis indicates the amount of Cerberus 1 (pM).

As shown in Fig. 3, in the activity evaluation using DE differentiation as the index, concentration-dependent activity was observed in the human activin A generated by furin protease cleavage, produced using a plant by the method of the present invention. In addition, the intensity of the activity was approximately 85% of the R&D 100 ng/mL with the addition in an amount of 1/100.

### 9. Evaluation of Activity by Smad Reporter Assay

The intensity of the Smad signal activated by activin A was quantified using Cignal Smad reporter assay (QIAGEN) using the human activin A generated by the recombinant activin A precursor protein and the furin protease cleavage, produced by the method of the present invention.

First, to 25 µL of the Opti-MEM medium, 0.6 µL of the Attractene reagent (QIAGEN) was added, followed by incubation at room temperature for 5 minutes. To the above-mentioned mixed solution, a mixed solution of 25 µL of the Opti-MEM medium and 1 µL of the Smad reporter vector(QIAGEN) was added, followed by incubation at room temperature for 20 minutes. This mixed solution was added to the Collagen I-coated 96-well plate (IWAKI), and HEK293E cells at 40,000 cell/well were sowed and incubated in a 5% CO₂ incubator at 37°C overnight to conduct transfection of the Smad reporter vector.

After the transfection was completed, all the culture supernatant was removed. An Opti-MEM medium (starving medium) containing 0.5% FBS, 1% NEAA, and penicillin-streptomycin was added in an amount of 75 µL, followed by culturing at 37°C for 4 hours to make the cells starved.

The human activin A or the recombinant activin A precursor protein produced by the method of the present invention or a starving medium to which activin A produced by R&D systems was added such that the concentration became twice the final concentration as control was added in an amount of 75 µL to cells, followed by culturing in a 5% CO₂ incubator at 37°C overnight to stimulate the cells. The human activin A produced by the method of the present invention was evaluated using a partially purified mixed solution obtained by mixing furinΔC purified through the Co column and the recombinant activin A precursor protein comprising the African clawed frog or green sea turtle-derived prodomain in the same manner as in the above-described 8, and was used while being diluted at rates described in Fig. 4. The recombinant activin A precursor proteins synthesized in the plant by the method of the present invention were as generated as described above, the recombinant activin A precursor protein comprising the zebrafish-derived prodomains used the protein extracts purified through the Ni column, and the other recombinant activin A precursor proteins used the protein extracts purified through the Co column, and these were used while being diluted at rates as described in Figs. 4 and 5. For the concentration of the recombinant activin A precursor protein in each extract, the concentration of the recombinant activin A precursor protein comprising the African clawed frog-derived prodomain was 362 ng/µL, the concentration of the recombinant activin A precursor protein comprising the green sea turtle-derived prodomain was 63 ng/µL, the concentration of the recombinant activin A precursor protein comprising the zebrafish-derived prodomain was 332 ng/µL, the concentration of the recombinant activin A precursor protein comprising the channel catfish-derived prodomain was 178 ng/µL, and the concentration of the recombinant activin A precursor protein comprising the coelacanth-derived prodomain was 114 ng/µL. The concentrations were obtained by the Bradford method after the protein purification.

The Dual-Luciferase Reporter Assay System (Promega) was used for the reporter assay. First, 100 µL of the medium supernatant was removed, 50 µL of the Glo reagent was added, and cells were dissolved at room temperature for 10 minutes. The activation of the Smad pathway was quantified by detecting the light emission of Firefly luciferase emitted from the cell solution using a plate reader. Subsequently, 50 µL of a Glo & Stop reagent solution was added, and the light emission of Renilla luciferase, which is an internal standard, was detected after 10 minutes to quantify the number of cells. In accordance with the Smad activity = (the light emission intensity of Firefly luciferase)/(the light emission intensity of Renilla luciferase), the Smad activity of each sample was quantified. The relative value of the Smad activity for the sample (Mock) to which no activin A was added was obtained as Relative Smad activity.

The results are shown in Figs. 4 and 5. Fig. 4 shows, in order from the left, the activin A produced by R&D systems, the recombinant activin A precursor protein comprising the African clawed frog-derived prodomain, the human activin A obtained by enzyme-cleaving the recombinant activin A precursor protein comprising the African clawed frog-derived prodomain, the recombinant activin A precursor protein comprising the green sea turtle-derived prodomain, the human activin A obtained by enzyme-cleaving the recombinant activin A precursor protein comprising the green sea turtle-derived prodomain, and the recombinant activin A precursor protein comprising the zebrafish-derived prodomain.

Fig. 5 shows, in order from the left, the activin A produced by R&D systems, the recombinant activin A precursor protein comprising the zebrafish-derived prodomain, the recombinant activin A precursor protein comprising the channel catfish-derived prodomain, and the recombinant activin A precursor protein comprising the coelacanth-derived prodomain.

From these results, it was revealed that all of the recombinant activin A precursor protein comprising the zebrafish-derived prodomain, the recombinant activin A precursor protein comprising the African clawed frog-derived prodomain, the recombinant activin A precursor protein comprising the green sea turtle-derived prodomain, the recombinant activin A precursor protein comprising the channel catfish-derived prodomain, and the recombinant activin A precursor protein comprising the coelacanth-derived prodomain had activity.

### Industrial Applicability

According to the present invention, it is possible to obtain an active protein without refolding even with a protein that is difficult to be folded such as activin. The method for producing human activin A of the present invention is expected to be utilized for the industrial production of human activin A.

In addition, the recombinant activin A precursor protein of the present invention has human activin A activity unlike the conventional findings, and is therefore expected to be utilized in various fields.

## Claims

1. A recombinant activin A precursor protein comprising:
a human mature activin A sequence; and
a non-human activin A prodomain, wherein
the recombinant activin A precursor protein is not a naturally occurring activin A precursor protein.

2. The recombinant activin A precursor protein according to claim 1, wherein the non-human activin A prodomain is an activin A prodomain of an amphibian, a reptile, or a fish.

3. The recombinant activin A precursor protein according to claim 1 or 2, wherein the non-human activin A prodomain is an activin A prodomain of a fish.

4. The recombinant activin A precursor protein according to claim 3, wherein the activin A prodomain of a fish comprises a sequence having an amino acid sequence identity of 90% or more with a sequence represented by SEQ ID NO: 1.

5. The recombinant activin A precursor protein according to claim 4, wherein the activin A prodomain of a fish has the sequence having an amino acid sequence identity of 90% or more with the sequence represented by SEQ ID NO: 1 at positions corresponding to positions 187 to 196 of a sequence represented by SEQ ID NO: 2.

6. The recombinant activin A precursor protein according to any one of claims 1 to 5, wherein the non-human activin A prodomain has an amino acid sequence identity of 90% or more with a sequence represented by SEQ ID NO: 2.

7. The recombinant activin A precursor protein according to any one of claims 1 to 5, wherein the non-human activin A prodomain has an amino acid sequence identity of 90% or more with a sequence represented by SEQ ID NO: 3.

8. The recombinant activin A precursor protein according to any one of claims 1 to 5, wherein the non-human activin A prodomain has an amino acid sequence identity of 90% or more with a sequence represented by SEQ ID NO: 4.

9. The recombinant activin A precursor protein according to claim 1 or 2, wherein the non-human activin A prodomain has an amino acid sequence identity of 90% or more with a sequence represented by SEQ ID NO: 5.

10. The recombinant activin A precursor protein according to claim 1 or 2, wherein the non-human activin A prodomain has an amino acid sequence identity of 90% or more with a sequence represented by SEQ ID NO: 6.

11. The recombinant activin A precursor protein according to any one of claims 1 to 10, comprising a sequence specifically cleavable by a protease between the human mature activin A sequence and the non-human activin A prodomain.

12. A polynucleotide encoding the recombinant activin A precursor protein according to any one of claims 1 to 11.

13. A recombinant vector comprising the polynucleotide according to claim 12.

14. A transformant comprising the polynucleotide according to claim 12 or the recombinant vector according to claim 13.

15. A method for producing the recombinant activin A precursor protein according to any one of claims 1 to 11, comprising a step of, in a plant cell into which the polynucleotide according to claim 12 or the recombinant vector according to claim 13 has been introduced, or in a plant comprising the plant cell, expressing the polynucleotide.

16. A method for producing human activin A comprising the steps of:
in a plant cell into which a polynucleotide encoding the recombinant activin A precursor protein according to claim 11 or a recombinant vector comprising the polynucleotide has been introduced, or in a plant comprising the plant cell, expressing the polynucleotide to generate the recombinant activin A precursor protein; and
treating the recombinant activin A precursor protein with the protease.
